# EUROPÄISCHE PATENTSCHRIFT

(11) **EP 1 051 197 B1**
(45) Veröffentlichungstag und Bekanntmachung des Hinweises auf die Patenterteilung: **10.11.2004**
(21) Anmeldenummer: 99902446.6
(22) Anmeldetag: 02.02.1999
(51) Int. Cl.: A61L 2/08, A23L 3/26, A23B 4/015, A23B 5/015, A23B 7/015, A23B 9/06, A23L 3/00

(54) **VERFAHREN ZUM BESTRAHLEN EINES GUTES MIT ELEKTRONENBESTRAHLUNG**
METHOD FOR IRRADIATING AN ITEM BY ELECTRON BEAM RADIATION
PROCEDE D'IRRADIATION D'UN MATERIAU AVEC FAISCEAUX D' ELECTRONS

(30) Priorität: 05.02.1998 AT 20898
(43) Veröffentlichungstag der Anmeldung: 15.11.2000
(73) Patentinhaber: Mediscan GmbH & Co. KG, 4550 Kremsmünster (AT)
(72) Erfinder: BIERBAUMER, Hans-Peter, A-4532 Rohr (AT)
(74) Vertreter: Secklehner, Günter
(86) Internationale Anmeldenummer: PCT/AT1999/000027
(87) Internationale Veröffentlichungsnummer: WO 1999/039750

(56) Entgegenhaltungen:
- EP-A- 0 731 626
- SADAT T. & VASSENAIX M.: "Use of a linear accelerator for decontamination of deboned poultry meat" RADIATION PHYSICS AND CHEMISTRY, Bd. 36, Nr. 5, 1990, Seiten 661-665, XP002111443
- Z.ZIMEK ET AL.: "EB industrial facility for radiation sterilization of medical devices" RADIATION PHYSICS AND CHEMISTRY, Bd. 42, Nr. 1-3, 1993, Seiten 571-572, XP002111442
- ALLEN J T ET AL: "A fully integrated 10 MeV electron beam sterilization system" RADIATION PHYSICS AND CHEMISTRY, Bd. 46, Nr. 4, 12. Oktober 1995 (1995-10-12), Seite 457-460 XP004051111 ISSN: 0969-806X
- Proceedings of the 10th International Meeting on radiation Processing, 11-16 May 1997, Anaheim, USA, veröffentlicht in "Radiation Physics and Chemistry", Vol 52,Nr 1-6 (1998-06-01), seite 491-494. Hackett J L: "A state of the art electron beam sterilization facility - An integrated system" XP002112487
- Proceedings of the 10th International Meeting on radiation Processing, 11-16 May 1997, Anaheim, USA, veröffentlicht in "Radiation Physics and Chemistry", Bd 52,Nr 1-6 (1998-06-01), seite 469-473. Kamino Y: "10 MeV 25kW industrial electron LINAC" XP002112515
- MCKEOWN J ET AL: "Beam scanning for dose uniformity" RADIATION PHYSICS AND CHEMISTRY, Bd. 46, Nr. 46, 12. September 1995 (1995-09-12), Seite 1363-1372 XP004051299 ISSN: 0969-806X
- MCLAUGHLIN W L ET AL: "Dosimetry systems for radiation processing" RADIATION PHYSICS AND CHEMISTRY, Bd. 46, Nr. 46, 12. September 1995 (1995-09-12), Seite 1163-1174 XP004051261 ISSN: 0969-806X
- BURNS P ET AL: "The measurement, control, and validation of critical parameters in an electron beam sterilization facility" NUCLEAR INSTRUMENTS & METHODS IN PHYSICS RESEARCH, SECTION - B: BEAM INTERACTIONS WITH MATERIALS AND ATOMS, Bd. 113, Nr. 1, 1. Juni 1996 (1996-06-01), Seite 96-98 XP004007773 ISSN: 0168-583X

## Beschreibung

Die Erfindung betrifft ein Verfahren zur Bestrahlung eines Gutes gemäß den Merkmalen des Anspruches 1, sowie eine Anlage zum Bestrahlen eines Gutes gemäß Anspruch 19.

Die strahlenchemische Behandlung von Produkten unterschiedlicher Spezies mit Strahlung aus den verschiedensten Quellen, z.B. γ-, Elektronenstrahlung, ist ein Verfahren, welches in den letzten Jahren immer mehr an Bedeutung gewinnt. Durch den Einsatz hochenergetischer Strahlung kann dieses Verfahren auch zur Sterilisation von Produkten eingesetzt werden. Bislang kam dabei aber vor allem zur Erreichung dieser Qualitätssteigerung neben der Begasung mit Giften, wie Methylbromid oder Ethylenoxid, vor allem die Bestrahlung mit radioaktiven Isotopen, beispielsweise Kobalt 60 oder Cäsium 137, zum Einsatz. Zwar werden durch derartige Verfahren Mikroben, Bakterien und Keime in hohem Maße abgetötet, es ist jedoch dabei zu bedenken, daß die Strahlenquelle ständig aktiviert ist und nicht "einfach" abgeschaltet werden kann, sodaß bei Wartungsarbeiten in diesen Anlagen ständig eine potentielle Gefährdung des Wartungspersonals gegeben ist. Zudem ist diese Art von Strahlenkosmetik zumindest in der Welt der Medien nicht unumstritten und lassen sich derart behandelte Produkte nur schlecht verkaufen. Außerdem gehen wichtige Vitamine, Mineralien und Nährwerte verloren. Eine Bestrahlungsanlage dieser Art ist beispielsweise aus der US 3,564,241 A bekannt. Dieses Dokument behandelt eine Anlage, bei der Waren aller Art zur Sterilisation in Körbe geladen werden, welche auf über Kopf angebrachten Schienen geführt sind. Die Körbe werden auf diesen Schienen in das Innere einer Strahlungskammer verbracht, wo sie durch die gewählte Art der Verlegung der Führungsschiene sowohl von vorne als auch von hinten mit Kobalt 60 bestrahlt werden.

Um diese Probleme zu vermeiden, wurden die Bestrahlungsverfahren derart weiterentwickelt, daß anstelle von radioaktivem Material beheizte Kathoden als Strahlenquelle zum Einsatz kamen. Die aus der Kathode austretenden Elektronen werden zur Anode hin beschleunigt und fokusiert und im Endeffekt auf die zu sterilisierenden Produkte gelenkt. Die Praxis zeigt, daß der Sterilisationsgrad der Produkte mit jenem vergleichbar ist, der durch radioaktive Strahlenbehandlung erzielt werden kann. Eine derartige Anlage ist aus der WO 94/22162 A bekannt. In ihrem Aufbau ist diese Anlage, abgesehen von der Art der Strahlenquelle, mit der aus oben angeführter US-A bekannten Anlage vergleichbar. Auch hier werden wiederum die Waren in Körben, welche auf einer Schiene geführt sind, durch den Behandlungsraum transportiert. Nachteilig bei dieser Art der Beförderung der Produkte ist es, daß durch die gewählte Art des Transportsystems, im speziellen Teile der Transportkörbe, welche einer ständig wiederkehrenden Bestrahlung ausgesetzt sind, dieses einem erhöhten Verschleiß unterliegt.

In "Radiation Physics and Chemisty" Bd. 36, Nr.5 Seiten 661 - 665 (1990) wird das Einsetzen einer Elektronenbeschleunigungsanlage zur Sterilisation beschrieben.

Der vorliegenden Erfindung liegt die Aufgabe zugrunde, ein Verfahren sowie eine Anlage zum Bestrahlen eines Gutes zu schaffen, die eine gleichmäßige Beaufschlagung mit Elektronen über einen möglichst großen Volumensbereich ermöglicht.

Diese Aufgabe der Erfindung wird durch die Merkmale des Anspruches 1 gelöst. Vorteilhaft ist dabei, daß es mit dem derartigen Verfahren erstmals möglich ist, Güter unterschiedlichster Art, beispielsweise Lebensmittel wie Gewürze, Wasser oder dgl., Werkstoffe wie z.B. Kunststoffe, Keramiken, Metalle oder dgl., so zu bestrahlen, daß damit eine bedeutende Qualitätsverbesserung erreicht werden kann. Von Vorteil ist dabei auch, daß die Elektronenstrahlung mit einer vordefinierbaren Frequenz gepulst werden kann, wodurch die Homogenität der Dosisverteilung im zu bestrahlenden Gut günstig beeinflußt werden kann.

Durch die Ausbildung nach Anspruch 2 wird auf vorteilhafte Weise eine weitere Steigerung der Homogenität der Dosisverteilung in dem zu bestrahlenden Gut erreicht.

Mit der Ausgestaltung nach Anspruch 3 ist es auf vorteilhafte Weise möglich, die jeweils erforderliche Strahlendosis auf das zu bestrahlende Gut und im speziellen auf dessen Abmessungen abzustimmen.

Durch die Ausbildung nach Anspruch 4 ist es auf vorteilhafte Weise möglich, bei einer hohen Produktionsrate die zu verabreichende Dosis auf die jeweiligen Eigenschaften der zu bestrahlenden Güter abzustimmen.

Durch vorteilhafte Weiterbildungen nach den Ansprüchen 5 und 6 wird ein hoher Automatisierungsgrad des Bestrahlungsprozesses erreicht.

Gemäß einer Ausbildung nach Anspruch 7 können die zu bestrahlenden Güter bzw. die eingesetzten Transportelemente auf einfache Art individualisiert werden und ist so ein Nachvollzug des Bestrahlungsprozesses zu einem späteren Zeitpunkt auf einfache Weise möglich.

Dabei erweist sich eine Ausgestaltung nach Anspruch 8 als vorteilhaft, da damit mit einem hohen Sicherheitsgrad ein zur Kontrolle der verabreichten Strahlendosis verwendetes Dosimeter eindeutig dem Gut, an dem dieses Dosimeter angebracht war, zugeordnet werden kann.

Nach den vorteilhaften Weiterbildungen gemäß den Ansprüchen 9 und 10 wird eine Produktivitätssteigerung erreicht.

Durch die Ausgestaltung nach Anspruch 11 ist es auf vorteilhafte Weise möglich, Güter entsprechend ihrem Behandlungsgrad dem Beschleunigerraum zuzuführen.

Dabei erweist sich eine Ausgestaltung nach Anspruch 12 als vorteilhaft, da damit eine weitere Steigerung des Automatisierungsgrades des Produktionsprozesses erfolgen kann.

Nach einer Ausführungsvariante gemäß Anspruch 13 können fertigbestrahlte Güter einer Verladezone zugeführt werden.

Die Ausgestaltung nach Anspruch 14 stellt eine wirkungsvolle Maßnahme zur Verfügung, mit der die im Gut deponierte Dosis definiert werden kann.

Bei der Ausgestaltung nach Anspruch 15 ist von Vorteil, daß die zu verabreichende minimale Strahlendosis über signifikante Punkte im bzw. am Gut ermittelt wird, sodaß die im eigentlichen Produktionsprozeß verwendeten Parameter für die Bestrahlung die größtmögliche Gutschonung gewährleisten und wonach die Verfahrensparameter in Abhängigkeit von der zu verabreichenden Strahlendosis eingestellt, geregelt und kontrolliert werden können.

Vorteilhaft ist auch eine Ausführungsform nach Anspruch 16, wonach ein Mittel zur Verfügung gestellt wird, welches eine wiederkehrende Kontrolle der Stabilität des Bestrahlungsprozesses ermöglicht.

Durch die Weiterbildung nach Anspruch 17 kann die Kontrolle der Stabilität des Bestrahlungsprozesses individuell und zufallsverteilt erfolgen.

Es ist aber auch eine Weiterbildung des Verfahrens, wie in Anspruch 18 beschrieben, möglich, bei der die Kontrolle des Bestrahlungsprozesses durch z.B. Visualisierung auf einem Bildschirm praktisch auf einen Blick und die Regelung einzelner Anlageteile, insbesondere des Prozeßförderers und des Elektronenbeschleunigersystems mit kurzer Zeitverzögerung erfolgen kann.

Die Aufgabe der Erfindung wird aber auch durch die Merkmale im Anspruch 19 gelöst. Dabei erweist sich als vorteilhaft, daß im Vergleich zu herkömmlichen Anlagen die Beschränkung auf eine bestimmte maximale Gutgröße weitestgehend aufgehoben ist und daß es zudem möglich ist, den Elektronenbeschleuniger und den größten Teil des Fördersystems in unterschiedlichen Räumen anzuordnen. Damit werden Teile des Fördersystems bestmöglich vor einer zu hohen Strahlenbelastung geschützt.

Durch eine Anordnung der Beschleunigereinheit gemäß Anspruch 20 ist eine vorteilhafte Reduzierung der Beschaffungskosten einer derartigen Anlage zu erzielen, da jener Raum, in dem der Elektronenbeschleuniger angeordnet ist, in seinen Abmessungen klein gehalten werden kann und insbesondere bei unterirdischer Anordnung das umliegende Erdreich zur Abschirmung der Elektronenstrahlung herangezogen werden kann.

Vorteilhaft ist weiters eine Ausbildung nach Anspruch 21, da damit eine Kontaminierung der fertig bestrahlten Güter weitestgehend ausgeschlossen werden kann.

Nach einer anderen Ausführungsvariante gemäß Anspruch 22 können Elektronen auf einfache Art auf eine hohe Geschwindigkeit beschleunigt werden und steht somit immer ein ausreichendes Depot an Elektronen hoher Energie zur Verfügung.

Gemäß einer Ausbildung, wie im Anspruch 23 beschrieben, können die zu bestrahlenden Güter in nur einem Umlauf endgefertigt werden. Gleichzeitig ist es möglich, eine derartige Elektronenaustrittsvorrichtung zur Beschleunigung der Elektronen heranzuziehen.

Von Vorteil ist auch eine Ausbildung nach Anspruch 24, da damit Güter mit großen Abmessungen bestrahlt werden können, ohne daß Qualitätseinbußen der Bestrahlung zu erwarten sind.

Von Vorteil sind aber auch Ausbildungen nach Anspruch 25, da damit eine homogene Verteilung der deponierten Strahlendosis erreicht werden kann.

Vorteilhaft ist auch eine weitere Ausführungsform nach Anspruch 26, wonach es möglich wird, das zu bestrahlende Gut erneut an der Elektronenaustrittsvorrichtung zutransportieren.

Nach den Ausführungsformen gemäß dem Anspruch 27 wird erreicht, daß bei konstanten Betriebsparametern für die Beschleunigereinheit die Homogenität der verabreichten Dosis im Gut großteils gewährleistet werden kann.

Gemäß einer Ausbildung nach Anspruch 28 ist es möglich, das Fördersystem aus kostengünstigen und wartungsfreundlichen Einzelbauteilen zusammenzustellen.

Die Aufteilung des Gesamtfördersystems in einzelne Teile, wie in Anspruch 29 beschrieben, ermöglicht mit Vorteil, diese Teile auf die jeweilige Strahlenbelastung durch den Elektronenbeschleuniger abzustimmen.

Vorteilhaft bei einer Ausgestaltung nach Anspruch 30 ist, daß durch das Anbringen von zumindest einer Positionserkennungsvorrichtung es möglich ist, die erfindungsgemäße Anlage weitestgehend vollautomatisch zu betreiben.

Durch den Einsatz eines Querverfahrwagens als Querförderer nach Anspruch 31 ist es möglich, eine kostengünstige Variante für diesen Teil des Fördersystems zur Verfügung zu stellen, da damit sowohl der Zu- als auch der Abtransport der Güter zu und aus dem Prozeßraum möglich ist.

Nach einer Ausführungsvariante gemäß Anspruch 32 ist es mit Vorteil möglich, die Bewegung der Güter durch den Elektronenstrahl auf die jeweiligen Erfordernisse abzustimmen.

Vorteilhaft ist auch eine Ausbildung nach Anspruch 33, womit ein einfaches Entfernen von fertigbestrahlten Gütern aus dem Bestrahlungsprozeß ermöglicht wird.

Durch die Weiterbildungen nach den Ansprüchen 34 bis 36 ist mit Vorteil eine Individualisierung der Güter zu erreichen, sodaß damit ein wirkungsvolles Mittel für das Qualitätsmanagement zur Verfügung steht.

Dabei erweisen sich Ausgestattungen nach den Ansprüchen 37 und 38 als vorteilhaft, wonach die Individualität der Güter automatisch erfaßt werden kann.

Gemäß den Ausbildungen nach den Ansprüchen 39 und 40 wird ein Unterbrechen des Gutstromes durch Fluktuationen im Aufgabebereich weitestgehend aufgehoben.

Nach den vorteilhaften Weiterbildungen gemäß den Ansprüchen 41 und 42 ist eine weitere Steigerung des Automatisierungsgrades der erfindungsgemäßen Anlage möglich und wird zudem eine hohe Betriebssicherheit der Automatisierung erreicht. Mit derart ausgebildeten Betätigungseinrichtungen für einen Gutstop wird eine nicht wartungsintensive und im Dauerbetrieb stabile Variante zur Verfügung gestellt.

Gemäß den Ausbildungen nach den Ansprüchen 43 und 44 ist es mit Vorteil und auf einfach Weise möglich, den Behandlungsgrad der Güter festzustellen und somit den Wirkungsgrad der Gesamtanlage zu verbessern.

Die Erfindung wird im nachfolgenden anhand der in den Zeichnungen dargestellten Ausführungsbeispiele näher erläutert.

Es zeigen:
- Fig. 1: einen schematischen und stark vereinfachten Grundriß der Anlagenteile zur Erzeugung und Beschleunigung der Elektronen;
- Fig. 2: einen Grundriß in schematischer und vereinfachter Darstellung einer erfindungsgemäßen Anlage;
- Fig. 3: eine Ausführungsvariante des Fördersystems für eine erfindungsgemäße Anlage;
- Fig. 4: eine weitere Ausführungsform eines Fördersystems in Draufsicht;
- Fig. 5: eine Ausführungsvariante eines Fördersystems in Draufsicht und vereinfachter Darstellung mit mehrfachen Zufuhr- bzw. Abtransportmöglichkeiten für zu behandelnde Güter;
- Fig. 6: eine weitere Ausführungsvariante für ein Fördersystem für eine erfindungsgemäße Anlage;
- Fig. 7: eine Ausführungsvariante für eine erfindungsgemäße Anlage in Draufsicht mit getrennten Bereichen für die Zufuhr und den Abtransport von zu behandelnden Gütern;
- Fig. 8: eine erfindungsgemäße Anlage in Draufsicht mit zusätzlich gekapseltem Bestrahlungsraum;
- Fig. 9: eine Ausführungsvariante einer erfindungsgemäßen Anlage in Stirnansicht geschnitten mit senkrecht angeordneten Labyrinthen für die Zufuhr bzw. den Abtransport von zu bestrahlenden Gütern;
- Fig. 10: eine Ausführungsvariante einer Elektronenaustrittsvorrichtung in vereinfachter, schematischer Darstellung.

Einführend sei festgehalten, daß in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind bei einer Lageänderung sinngemäß auf die neue Lage zu übertragen.

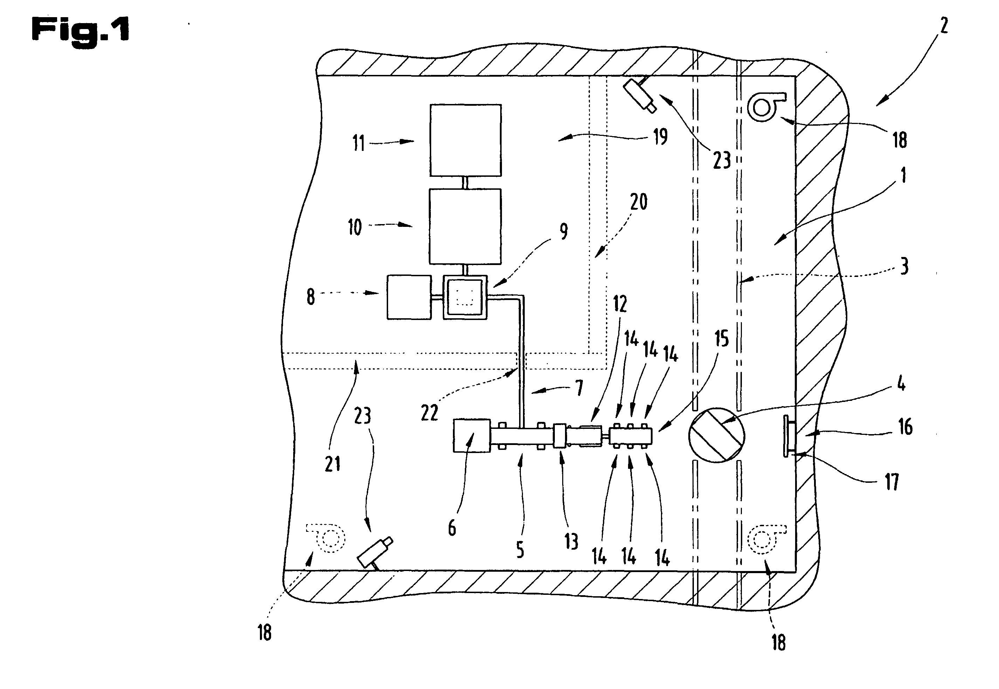

Fig. 1 zeigt im Grundriß und schematisch vereinfacht einen Bestrahlungsraum 1 einer erfindungsgemäßen Anlage 2. Zur Vereinfachung und übersichtlicheren Darstellung sind jene Anlagenteile, welche sich oberhalb eines durch eine Abdeckung des Bestrahlungsraumes 1 gebildeten Niveaus befinden, nicht bzw. strichpunktiert dargestellt. Diese Anlagenteile betreffen vornehmlich ein Fördersystem 3.

Die dargestellten Anlagenteile sind bevorzugt unterirdisch angeordnet, womit vorteilhafterweise erreicht wird, daß jene Anlagenteile, welche sich oberhalb eines durch eine Abdeckung des Bestrahlungsraumes 1 gebildeten Niveaus befinden, einer geringen Strahlenbelastung ausgesetzt sind. Da aufgrund dessen das den Bestrahlungsraum 1 umgebende Erdreich als Strahlenschutzkörper ausgenutzt werden kann, ist es möglich, die überirdisch plazierten Bauwerkteile, wie z.B. die Wände, in ihren Dimensionen gegenüber herkömmlichen, vergleichbaren Anlagen geringer auszuführen, wodurch sich unter anderem die Wirtschaftlichkeit der erfindungsgemäßen Anlage 2 steigern läßt.

Zur Bestrahlung von Gütern 4 werden vornehmlich schnellbewegte Elektronen verwendet. Diese werden bevorzugt in einem Linearbeschleuniger 5 von einer Glühkathode 6 freigesetzt und in Richtung einer Anode aufgrund der vorherrschenden Potentialdifferenz vorbeschleunigt. Mit dem bevorzugt verwendeten Linearbeschleuniger 5 sind Leistungen zwischen 5 kW und 40 kW, bevorzugt zwischen 10 kW bis 30 kW, insbesondere 20 kW, zu erreichen, wobei zur Bestrahlung der Güter 4 Elektronen mit vorzugsweise einer Energie zwischen 5 MeV und 30 MeV, bevorzugt 10 MeV, verwendet werden.

Selbstverständlich ist es möglich, mehrere Elektronenbeschleunigereinheiten auch unterschiedlicher Bauart in der Anlage 2 zu verwenden, um z.B. eine Mehrfachbestrahlung von mehreren Seiten zu erreichen. Zum besseren Verständnis wird allerdings in der folgenden Beschreibung auf diese Möglichkeit nicht näher eingegangen und soll diese aber vom Schutzumfang nicht ausgeschlossen sein.

Da aufgrund dieser hohen Energien die Beschleunigung mit elektrostatischen Feldern, wie sie beispielsweise in herkömmlichen Bildröhren mit einer Energie von ca. 30 kV verwendet werden, nicht möglich ist, werden zur endgültigen Beschleunigung der Elektronen vorzugsweise elektromagnetische Wellen verwendet. Zur Erzeugung dieser elektromagnetischen Wellen entlang der Beschleunigerstrecke können z.B. Hohlraumresonatoren 7, sogenannte Wave Guids, verwendet werden. In diesen Hohlraumresonatoren 7, die beispielsweise aus Metallen wie Kupfer, aus Kupferlegierungen wie Bronze, Messing, aus Stahl, Keramik, Kunststoff oder dgl., gearbeitet sein können, bildet sich eine stehende Welle aus, deren Feldverteilung durch die Geometrie der Hohlraumresonatoren 7 und der Frequenz einer eingespeisten Mikrowellenenergie bestimmt wird.

Die stehende Welle in der Beschleunigereinheit wird von bevorzugt gepulsten Mikrowellen angeregt, welche beispielsweise von einem Oszillator 8, einem sogenannten RF-Driver, erzeugt und durch einen Mikrowellenverstärker 9, beispielsweise einem Klystron oder dgl., verstärkt werden. Die dazu notwendige Energie liefert ein Hochspannungsmodulator 10, der von einer primären Energiequelle 11, beispielsweise dem öffentlichen Stromnetz, versorgt wird.

Die Frequenz der verwendeten Mikrowellen liegt im GHz-Bereich, wohingegen die aus dem Linearbeschleuniger 5 austretenden Elektronen eine Pulsfrequenz von ca. 300 Pulsen pro Sekunde aufweisen.

Es sei an dieser Stelle erwähnt, daß selbstverständlich auch sämtliche anderen dem Stand der Technik entsprechende Methoden zur Beschleunigung der Elektronen verwendet werden können. Vor allem ist die Ausbildung einer Anlage 2 nach der Erfindung nicht beschränkt auf die Verwendung von Mikrowellen als Beschleunigungsmedium, welche von einem Klystron verstärkt werden.

Die derart beschleunigten, vorzugsweise mit einem Quadrupol 12 fokusierten Elektronen beaufschlagen, sofern ein Shutter 13 geöffnet ist, mit Hilfe von Ablenkmagneten 14 in einem definierten, vorbestimmten Verlauf nach ihrem Austritt aus einer Elektronenaustrittsvorrichtung 15, beispielsweise einem Scan-Horn mit bevorzugt rechteckigem Querschnitt, einer ringförmigen Elektronenaustrittsvorrichtung 15 oder dgl., die Güter 4. Die Güter 4 werden dabei, wie nachstehend noch näher erläutert wird, mit Hilfe eines Fördersystems 3 durch den aus der Elektronenaustrittsvorrichtung 15 austretenden Elektronenstrahl bewegt. Der Shutter 13 dient vor allem zur Verhinderung einer Beschädigung der inneren Bestandteile des Linearbeschleunigers 5 für den Fall, daß das Elektronenaustrittsfenster im Bereich der Elektronenaustrittsvorrichtung 15 beschädigt wird.

Die Dimensionen der Elektronenaustrittsvorrichtung 15 sind vorzugsweise so gewählt, daß eine Scanhöhe bis zu 100 cm, bevorzugt 60 cm, erreicht werden kann.

Vorzugsweise treten aus der Elektronenaustrittsvorrichtung 15 gepulste Elektronen aus und ist es möglich, in bezug auf die Güter 4 bzw. das Fördersystem 3 eine Pulsüberlappung von zumindest 30 %, vorzugsweise 50 %, einzustellen.

Es soll an dieser Stelle nicht unerwähnt bleiben, daß es möglich ist, mehrere Elektronenaustrittsvorrichtungen 15 einer Elektronenbeschleunigereinheit zuzuordnen.

Aus Gründen der Sicherheit kann in Fluchtrichtung des Linearbeschleunigers 5 hinter den Gütern 4, bevorzugt an einer Wand 16, ein sogenannter Beam-Stop 17, der bevorzugt aus einer Metallplatte mit hohem Elektroneneinfangquerschnitt, beispielsweise einer Aluminiumplatte, besteht, angebracht sein, sodaß eventuell durchschlagende Elektronen abgebremst werden und dabei nur eine weiche γ-Strahlung entsteht.

Da der Bestrahlungsraum 1 für Wartungsarbeiten von Menschen betreten werden können soll, kann zur Aufrechterhaltung eines verträglichen Klimas im Bestrahlungsraum 1 zumindest eine Entlüftungsvorrichtung 18, beispielsweise ein Ventilator oder dgl., vorgesehen werden, sodaß etwaige Reaktionsprodukte, welche durch die ionisierende Strahlung aus der Umgebungsluft gebildet werden, beispielsweise Ozon, aus dem Bestrahlungsraum 1 verbracht werden. Wie dies anhand der strichlierten Entlüftungsvorrichtungen 18 in Fig. 1 gezeigt ist, können bei der erfindungsgemäßen Anlage 2 weitere Entlüftungsvorrichtungen 18 vorgesehen sein.

Selbstverständlich können zur Beschleunigung der Elektronen auch sämtliche dem Stand der Technik entsprechende Beschleunigereinheiten verwendet werden. So ist beispielsweise der Einsatz von Ringbeschleunigern möglich.

Der Bestrahlungsraum 1 ist bevorzugt von Wänden begrenzt, beispielsweise aus Stahlbeton, Metall oder dgl., mit welchen es möglich ist, die Umgebung vom Bestrahlungsraum 1 abzuschirmen. Ebenso können die Bodenplatte bzw. die Abdeckung des Bestrahlungsraumes 1 aus diesen Materialien gefertigt sein. Da der Bestrahlungsraum 1 vorzugsweise unterirdisch angeordnet ist, können, wie bereits erwähnt, diese Wände bzw. die Bodenplatte und die Abdeckung bezüglich ihrer Dickenabmessungen geringer ausgeführt sein, da das umliegende Erdreich ebenfalls zur Abschirmung ausgenutzt werden kann.

Aufgrund der Lärmentwicklung des Oszillators 8 bzw. des Mikrowellenverstärkers 9 ist es von Vorteil, wenn diese Module der erfindungsgemäßen Anlage 2 in einem Raum 19 plaziert sind, welcher durch Wände 20, 21, beispielsweise aus Stahlbeton, vom Bestrahlungsraum 1 abgegrenzt sein kann. Die Verbindung zwischen dem Linearbeschleuniger 5 und dem Mikrowellenverstärker 9 durch den Hohlraumresonator 7 kann in diesem Fall über einen Durchbruch 22 in der Wand 21 erfolgen.

Selbstverständlich ist es in diesem Zusammenhang auch möglich, weitere Maßnahmen zur Senkung des Lärmpegels zu setzen. Beispielsweise können einzelne lärmerzeugende Anlagenteile gesondert abgeschirmt werden. Ebenso besteht natürlich die Möglichkeit, die Wände 20, 21 beispielsweise mit Lärmschutzplatten zu versehen.

Für die Fernüberwachung ist es von Vorteil, wenn im Bestrahlungsraum 1 zumindest eine Überwachungskamera 23 angebracht ist, welche mit einer Bedienungszentrale für die Anlage 2 in Verbindung steht. Dadurch wird es möglich, etwaige Unregelmäßigkeiten, beispielsweise im Bereich des Fördersystems 3, ohne Verzug zu erkennen und darauf in angemessener Art und Weise zu reagieren.

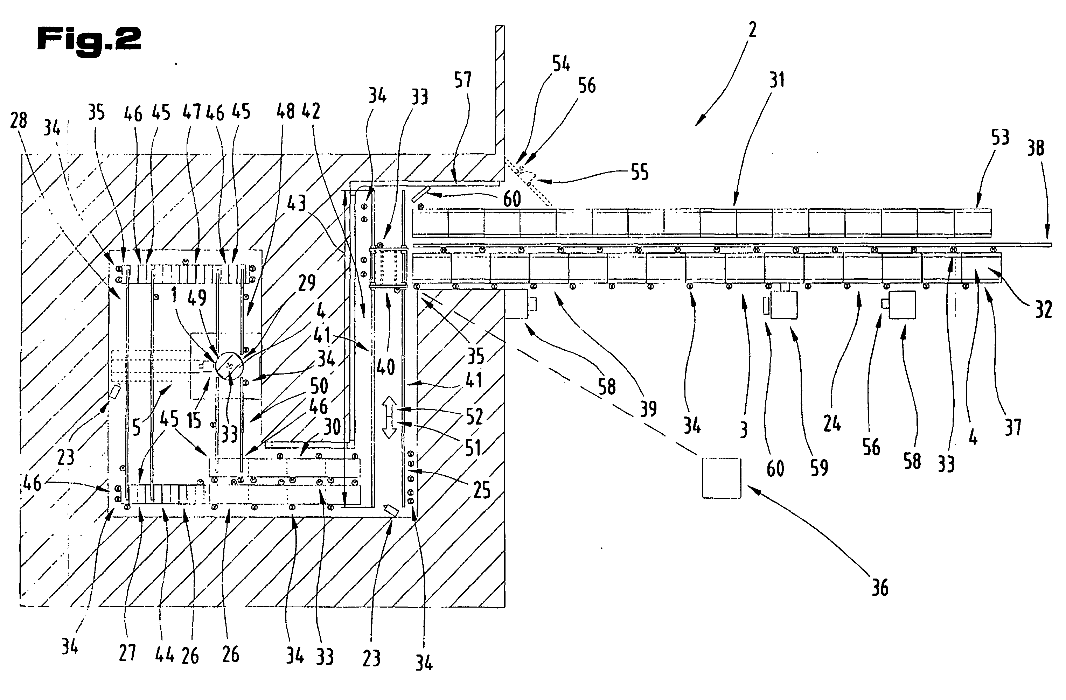

Fig. 2 zeigt einen Grundriß der erfindungsgemäßen Anlage 2 in schematischer und vereinfachter Darstellung. Dabei wurde zur Steigerung der Übersichtlichkeit auf jene Anlagenteile verzichtet, welche unterhalb des durch die Abdeckung des Bestrahlungsraumes 1 gebildeten Niveaus angeordnet sind, mit Ausnahme des andeutungsweise gezeigten Linearbeschleunigers 5 (in Fig. 2 zum Teil strichliert dargestellt).

Das aus Fig. 2 ersichtliche Fördersystem 3 ist bevorzugt in einen Aufgabepuffer 24, einen Querförderer 25, einen Zuführpuffer 26 in einem Prozeßraum 27, einen Stetigförderer 28, einen Prozeßförderer 29, einen Auslagerungspuffer 30 im Prozeßraum 27 sowie einen weiteren Puffer 31 unterteilt.

Ein Vorteil des in Fig. 2 dargestellten Fördersystems 3 ist, daß die Güter 4 auf bzw. in Transportelementen 32 dem Bestrahlungsraum 1 zugeführt werden, welche nicht ausschließlich dem Transport von Gütern 4 in der erfindungsgemäßen Anlage 2 dienen. Derartige Transportelemente 32 können beispielsweise Paletten, Transportboxen mit vollen bzw. durchbrochenen Seitenwänden oder dgl. jeweils aus beliebigen Materialien wie Holz, Kunststoff, Metall, etc. gebildet sein. Vorzugsweise werden sogenannte Europaletten mit den genormten Abmessungen der Aufstandsfläche 1200 mm x 800 mm verwendet, welche sich beispielsweise beim Transport von Gütern 4 auf der Straße durch den LKW bzw. auf der Schiene als Norm durchgesetzt haben. Verwendbar ist natürlich jedwedes andere Transportelement 32 mit beliebigen Abmessungen, insbesondere auch sogenannte Einwegpaletten.

Werden als Transportelement 32 Paletten verwendet, so kann das Leerpalettengewicht beispielsweise zwischen 1 kg und 30 kg, bevorzugt 20 kg, betragen. Zur optimalen Auslastung der erfindungsgemäßen Anlage 2, insbesondere des Linearbeschleunigers 5, kann das Vollpalettengewicht im Bereich zwischen 300 kg und 700 kg, bevorzugt 500 kg, liegen. Selbstverständlich sind diese Gewichtsangaben nur als Richtwerte zu verstehen, da eine minimale Gewichtsbeschränkung bei 0 kg anzusetzen ist und sich die maximale Gewichtsbeschränkung nach der jeweiligen Ausführung des Fördersystems 3 richtet.

Der Aufgabepuffer 24 kann aus Einzelantriebsplätzen, bevorzugt 15 Stück, bestehen. Jedem Einzelantriebsplatz ist dabei zumindest eine Antriebsvorrichtung 33, beispielsweise ein Motor, insbesondere ein Servomotor, zugeordnet. Zusätzlich kann an jedem Einzelantriebsplatz zumindest eine Positionserkennungsvorrichtung 34, beispielsweise eine Lichtschranke, ein elektrischer Sensor, ein Magnetsensor oder dgl., angebracht sein. Dadurch wird erreicht, da es sich bei dem in Fig. 2 dargestellten Förderprozeß im wesentlichen um einen diskontinuierlichen Prozeß handelt, daß, sobald ein Transportelement 32 mit Gütern 4 vom Aufgabepuffer 24 auf den Querförderer 25 übergeben wird, die Positionserkennungsvorrichtung 34 erkennt, daß zumindest ein Einzelantriebsplatz leer ist. Beispielsweise kann durch die Freigabe des Lichtschrankens 35 ein Signal an eine vorzugsweise zentrale Regel- und Steuereinrichtung 36 übergeben werden (in Fig. 2 andeutungsweise durch eine strichlierte Verbindung zwischen einer Lichtschranke und der Regel- und Steuereinrichtung 36 dargestellt), sodaß in der Folge die Antriebsvorrichtungen 33 in Gang gesetzt und somit die Transportelemente 32 um jeweils eine Position in Richtung Prozeßraum 27 versetzt werden. Damit wird ein Aufgabeplatz 37 frei und es kann ein weiteres Transportelement 32 dem Aufgabepuffer 24 zugeführt werden.

Der Aufgabeplatz 37 kann bevorzugt aus einem Hubtisch mit aufgebauter Rollenbahn bestehen. Damit wird mit Vorteil erreicht, daß Transportelemente 32 in einfacher Art und Weise, beispielsweise durch einen Stapler, dem Aufgabepuffer 24 zugeführt werden können.

Der Aufgabepuffer 24 kann durch eine Trennvorrichtung 38, beispielsweise eine Wand, einem Gitter oder dgl., vom Puffer 31 getrennt sein. Somit ist es möglich, den unreinen Aufgabebereich vom reinen Abnahmebereich zu separieren.

Am Aufgabepuffer 24 kann beispielsweise an einer Position 39 eine Expreßaufgabestelle für Transportelemente 32 eingebaut sein. Dadurch können einzelne Transportelemente 32 mit verkürzter Durchlaufzeit bearbeitet werden. Wird eine derartige Expreßaufgabestelle in Anspruch genommen, ist es möglich, daß die Regel- und Steuereinheit 36 den Transport von Gütern 4 auf dem Aufgabepuffer 24 bis zur Expreßaufgabestelle so lange unterbindet, bis letztere wieder frei ist. Erkannt kann dies - ebenso wie das Vorhandensein von Gütern 4 auf der Expreßaufgabestelle - z.B. automatisch mit den bereits erwähnten Positionserkennungsvorrichtungen 34 werden.

Vom Aufgabepuffer 24 werden die Transportelemente 32 dem Querförderer 25 übergeben. Dieser kann beispielsweise als Querverfahrwagen 40 ausgebildet sein. Dieser Querverfahrwagen 40 hat bevorzugt eine Rollenbahn aufgebaut und läuft auf Schienen 41. Damit ist es möglich, einzelne Transportelemente 32 zu übernehmen und diese durch ein Labyrinth 42 dem Zuführpuffer 26 zuzuführen.

Die Auswahl der Materialien für das Fördersystem 3 sowie deren Antriebsvorrichtungen 33 und Positionserkennungsvorrichtungen 34 werden vorzugsweise den strahlentechnischen Erfordernissen im Prozeßraum 27 bzw. Bestrahlungsraum 1 angepaßt. Dadurch wird mit Vorteil erreicht, daß ein weitestgehend störungsfreier Betrieb der erfindungsgemäßen Anlage 2 gewährleistet werden kann. Weiters können durch die entsprechende Auswahl der Materialien die Wartungsintervalle für einzelne Elemente der Anlage 2 verlängert werden.

Dem Querförderer 25 ist zumindest eine Antriebsvorrichtung 33 zugeordnet, sodaß dieser entlang einer Strecke 43 bewegt werden kann. Weiters können entlang dieser Strecke 43 in der Nähe des Querförderers 25 Positionserkennungsvorrichtungen 34 vorgesehen sein, welche wiederum mit der zentralen Regel- und Steuereinrichtung 36 verbunden sein können. Selbstverständlich ist es aber auch möglich, daß sämtliche Positionserkennungsvorrichtungen 34 jeweils eigenen, dezentralen Regel- und Steuereinrichtungen zugeordnet sind. Mit diesen Positionserkennungsvorrichtungen 34 wird es möglich, daß, wenn für den Querförderer 25 beispielsweise ein Querverfahrwagen 40 eingesetzt wird, dieser exakt an die einzelnen Positionen entlang der Strecke 43 zu bewegen ist, um die Übergabe von Transportelementen 32 zwischen den Teilen des Fördersystems 3, wie beispielsweise dem Aufgabepuffer 24 und dem Zuführpuffer 26 sowie dem Auslagerungspuffer 30 und dem Puffer 31, durchzuführen.

Auch aus Wirtschaftlichkeitsgründen ist es von Vorteil, da das in Fig. 2 dargestellte Fördersystem 3 ohnehin diskontinuierlich betrieben wird, wenn im Labyrinth 42 nur ein Querförderer 25 angeordnet ist und daß dieser sowohl die Zufuhr von Gütern 4 zum Prozeßraum 27 als auch deren Abtransport aus dem Prozeßraum 27 übernimmt. Dies schließt jedoch nicht aus, daß es auch möglich ist, getrennte Querförderer für die Zufuhr und den Abtransport von Gütern 4 im Labyrinth 42 anzuordnen. Um die Bewegung von Fördersystemteilen in zumindest zwei Richtungen zu ermöglichen, können an den entsprechenden Antriebsvorrichtungen 33 Frequenzumformer vorgesehen werden.

Die Kapazität des Querförderers 25 kann mit Vorteil so bemessen sein, daß die Zufuhr und der Abtransport von Gütern 4 zum bzw. aus dem Prozeßraum 27 in einer Zeit erfolgt, welche im Bereich zwischen 1 Min. und 10 Min., bevorzugt 3 Min., liegen kann. Damit wird eine optimale Abstimmung der Geschwindigkeit des Fördersystems 3 auf die Dauer der Bestrahlung von Gütern 4, welche sich bevorzugt auf Transportelementen 32 befinden, erreicht und somit die Wirtschaftlichkeit der Anlage 2 erhöht.

Vom Querförderer 25 werden die Transportelemente 32 an den Zuführpuffer 26 übergeben. Dieser kann wieder, wie in Fig. 2 andeutungsweise dargestellt, aus Einzelantriebsplätzen mit jeweils separaten Antriebsvorrichtungen 33 sowie Positionserkennungsvorrichtungen 34 aufgebaut sein. Andererseits ist es aber auch möglich, den Zuführpuffer 26 als Stetigförderer, beispielsweise Rollenförderer, Bandförderer, Kettenförderer, Stabförderer oder dgl., auszuführen. Möglich ist dabei auch, wie in Fig. 2 gezeigt, eine Kombination mehrerer unterschiedlicher Förderer für den Zuführpuffer 26, wie beispielsweise eine Kombination aus Einzelantriebsplätzen und einem Kettenförderer 44 bzw. Einzelrollenbahnen oder Rollenbahnen. Eine derartige Kombination verschiedener Förderer ist im übrigen für sämtliche entsprechende Elemente des Fördersystems 3 möglich.

Wie bereits erwähnt, können die einzelnen Antriebsvorrichtungen 33 und Positionserkennungsvorrichtungen 34 mit einer zentralen Regel- und Steuereinrichtung 36 verbunden sein, bzw. können diese Vorrichtungen dezentral einer Regelung und Steuerung unterliegen.

Als besonders vorteilhaft erweist sich der Einsatz von Kettenförderern im Prozeßraum 27, da diese Förderer weitestgehend unempfindlich gegenüber ionisierender Strahlung sind, sodaß, wie bereits erwähnt, die Wirtschaftlichkeit der Anlage 2 aufgrund der verlängerten Wartungsintervalle gesteigert werden kann. Daher wird als Stetigförderer 28 bevorzugt ein Kettenförderer 44 verwendet. Selbstverständlich können aber anstelle des Kettenförderers 44 jedwede andere Förderkomponenten als Stetigförderer 28 verwendet werden. Denkbar ist jedoch auch, daß anstelle des Stetigförderers 28 diskontinuierlich betriebene Förderer zum Einsatz kommen.

Um Niveauunterschiede zwischen dem Zuführpuffer 26 und dem Stetigförderer 28 auszugleichen bzw. um die Übergabe von Transportelementen 32 vom Zuführpuffer 26 auf den Stetigförderer 28 zu ermöglichen, kann bevorzugt an einem Übergabebereich 45 eine Senk- und Hebevorrichtung 46 für den Ecktransport angeordnet sein.

Selbstverständlich ist es auch möglich, den Stetigförderer 28, nicht, wie in Fig. 2 dargestellt, durchgehend auszuführen, sondern diesen aus mehreren Einzelförderern aufzubauen.

Vom Stetigförderer 28 werden die Transportelemente 32 an einen Förderer 47 übergeben, wobei im Übergabebereich 45 aus besagten Gründen wiederum eine Senk- und Hebevorrichtung 46 angeordnet sein kann. Diese Senk- und Hebevorrichtung 46 wird bevorzugt immer dann betätigt, wenn eine Positionserkennungsvorrichtung 34, beispielsweise eine Lichtschranke 35, durch das Transportelement 32 unterbrochen wird.

Der Förderer 47 ist bevorzugt ebenfalls als Stetigförderer ausgebildet und kann aus bereits besagten Komponenten, insbesondere aus einem Kettenförderer 44, aufgebaut sein.

In weiterer Folge werden die Transportelemente 32 in einem zweiten Übergabebereich 45 mit angeschlossener Senk- und Hebevorrichtung 46, welcher bevorzugt an der dem ersten Übergabebereich gegenüber liegenden Seite des Förderers 47 angeordnet ist, an einen Prozeßzuführförderer 48, welcher bevorzugt ebenfalls aus bereits besagten Komponenten aufgebaut ist, übergeben. Dieser Prozeßzuführförderer 48 verbringt die Transportelemente 32 zu einem Prozeßförderer 29.

Der Prozeßförderer 29 ist bevorzugt als Dreh-Hubtisch 49 ausgebildet. Dadurch wird es möglich, die zu bestrahlenden Güter 4 auf den Transportelementen 32 während einer Drehbewegung mit gleichzeitiger vertikaler Verschiebung des Dreh-Hubtisches 49 an der Elektronenaustrittsvorrichtung 15 des Linearbeschleunigers 5 vorbeizubewegen, sodaß die Bestrahlung mit dem Elektronenstahl erfolgen kann.

Dem Dreh-Hubtisch 49 ist wiederum jeweils zumindest eine Antriebsvorrichtung 33 und eine Positionserkennungsvorrichtung 34 zugeordnet, sodaß nach Erkennung des Vorhandenseins eines Transportelementes 32 auf dem Dreh-Hubtisch 49 dieser durch die Antriebsvorrichtung 33 in Bewegung gesetzt wird.

Auf dem Dreh-Hubtisch 49 können weitere Förderelemente, wie beispielsweise Transportrollen, Schienen oder dgl., angeordnet sein, wodurch eine rasche und einfache Positionierung des Transportelementes 32 auf dem Dreh-Hubtisch 49 möglich wird.

Selbstverständlich besteht aber auch die Möglichkeit, daß andere Kombinationen der Bewegungen des Dreh-Hubtisches 49 bzw. einzelne Bewegungsarten vorgesehen sind. So kann beispielsweise der Dreh-Hubtisch 49 um einen bestimmten Betrag in vertikaler Richtung, bevorzugt nach unten, bewegt werden, worauf anschließend eine Drehbewegung einsetzt, um die Güter 4 auf den Transportelementen 33 einer allseitigen Bestrahlung durch Elektronen auszusetzen. Dazu ist es von Vorteil, wenn entlang der Strecke der möglichen Vertikalbewegung des Dreh-Hubtisches 49 Positionserkennungsvorrichtungen 34 angebracht sind, sodaß eine zielgenaue Vertikalbewegung des Dreh-Hubtisches 49 möglich ist. Bevorzugt sind die Beträge der Vertikalbewegung des Dreh-Hubtisches 49 auf die maximale Höhe der Elektronenaustrittsvorrichtung 15 abgestimmt. Es ist jedoch auch möglich, daß die Vertikalbewegung des Dreh-Hubtisches 49 um solche Beträge erfolgt, daß daraus eine Überlappung der Bestrahlungsflächen (der Oberflächen der Güter 4) in bezug auf die maximale Scanhöhe resultiert. Damit können einerseits die Güter 4 einer mehrfachen Bestrahlung ausgesetzt werden, bzw. ist es andererseits möglich, den Linearbeschleuniger 5 mit Betriebsparametern zu verwenden, welche unterhalb der maximalen Belastungsgrenze des Linearbeschleunigers 5 liegen, sodaß ein schonendes Betreiben der erfindungsgemäßen Anlage 2 erfolgen kann.

Weiters ist es natürlich möglich, bei geeigneten Gütern 4, insbesondere bei sehr schmalen, stehenden Gütern 4, die Bewegung des Dreh-Hubtisches 49 nur in vertikaler Richtung auszuführen. Es können aber auch sämtliche anderen möglichen, hier nicht beschriebenen Bewegungsabläufe des Dreh-Hubtisches 49 durchgeführt werden.

Besonders vorteilhaft ist es, wenn die Güter 4 auf den Transportelementen 32 sowohl während des Absenkens als auch während des Anhebens auf das Niveau des Prozeßzuführförderers 48 bei gleichzeitiger Drehung des Dreh-Hubtisches 49 einer Bestrahlung durch Elektronen unterzogen werden, woraus wiederum eine Mehrfachbestrahlung mit den bereits beschriebenen Vorteilen bezüglich des Linearbeschleunigers 5 resultiert.

Als Vorteil kann es sich erweisen, wenn dem Dreh-Hubtisch 49 eine Positionserkennungsvorrichtung 34 zugeordnet ist, mit welcher bei unbeabsichtigter Unterbrechung des Bestrahlungsprozesses jene Stelle erkannt werden kann, für die noch von einer ausreichenden Bestrahlung ausgegangen werden kann. Ist es nämlich nicht möglich, daß die Antriebsvorrichtungen 33 des Prozeßförderers 29 einen sofortigen Stillstand von letzterem bewirken, so resultiert daraus in Bereichen der zuletzt bestrahlten Güter 4 ein ungenügender Behandlungsgrad. Mit den Daten aus der Positionserkennungsvorrichtung 34 kann nun der Prozeßförderer 29 bei wieder voll leistungsfähigem Linearbeschleuniger 5 mit der Antriebsvorrichtung 33 an besagte Position zurückgefahren werden und kann damit die Qualität des Bestrahlungsprozesses gewährleistet werden.

Am vorteilhaftesten ist es jedoch, wenn die Antriebsvorrichtung 33 des Prozeßförderers 29 einen sofortigen Stillstand ermöglicht, sobald ein Deffekt in der Beschleunigereinheit erkannt wird.

Sobald der Prozeßförderer 29 seine Be- und Entladeposition wieder erreicht hat, wird der Beschleuniger zur Erhöhung der Sicherheit der Anlage 2 abgeschaltet bzw. auf stand-by-Betrieb umgeschaltet. Dies ist jedoch kein zwingendes Erfordernis und dient, wie bereits gesagt, nur der Sicherheit der Anlage 2 bzw. der Vermeidung einer unnötigen Ionisierung der Atmosphäre im Bestrahlungsraum 1.

Vom Prozeßförderer 29 werden die Güter 4 auf den Transportelementen 32 an einen Prozeßabführförderer 50 übergeben, der aus den bereits beschriebenen Förderbausteinen des Fördersystems 3, insbesondere aus jenen des Prozeßzuführförderers 48, gebildet sein kann.

In der Folge können die Transportelemente 32 auf den Auslagerungspuffer 30 in einem Übergabebereich 45 mit der bereits beschriebenen Senk- und Hebevorrichtung 46 umgesetzt werden. Dieser Auslagerungspuffer 30, welcher bevorzugt für vier Transportelemente 32 ausgebildet sein kann (in Fig. 2 strichliert angedeutet), verbringt die Transportelemente 32 zum Querförderer 25. Am Querförderer 25 kann in der Folge beispielsweise der schon beschriebene Querverfahrwagen 40 die fertig bestrahlten Transportelemente 32 mit den Gütern 4 übernehmen und sie dem Puffer 31 zuführen.

Von besonderem Vorteil ist es, wenn die Logistik des Fördersystems 3, insbesondere des Querverfahrwagens 40, so abgestimmt wird, daß während einer Bewegungsrichtung gemäß Pfeil 51 Transportelemente 32 dem Zuführpuffer 26 zugeführt werden und daran anschließend bei einer Bewegung des Querverfahrwagens 40 gemäß Pfeil 52 fertigbestrahlte Güter 4 auf Transportelementen 32 vom Auslagerungspuffer 30 entfernt werden. Mit dieser Logistik kann die erforderliche Förderzeit des Querverfahrwagens 40 vorteilhafter Weise auf annähernd 50 % der maximal benötigten Förderzeit eingeschränkt werden.

Um eine weitgehende Automatisierung der erfindungsgemäßen Anlage 2 zu ermöglichen, können selbstverständlich entlang der Förderstrecke des Prozeßabführförderers 50 und des Auslagerungspuffers 30 die bereits erwähnten Elemente, wie Antriebsvorrichtungen 33 und Positionserkennungsvorrichtungen 34, angebracht sein.

Der Puffer 31 kann aus jedem beliebigen, dem Stand der Technik entsprechenden Stetigförderer gebildet sein, bzw. ist es möglich, diskontinuierliche Förderer zu verwenden. Bevorzugt besteht der Puffer 31 aus einer Schwerkraftbahn mit vorzugsweise sechzehn Plätzen, wobei der Platz Nr. 16 als flurebener Staplerabnahmeplatz 53 ausgebildet sein kann.

Anschließend ist es möglich, die Güter 4 auf den Transportelementen 32 einem Wickelapparat zuzuführen und mit einer Schrumpffolie oder dgl. zu versehen, wodurch versandfertige Einheiten entstehen.

Selbstverständlich ist es aber auch möglich, die Güter 4 nicht, wie beschrieben, in Transportelementen 32 sondern mit oder ohne Verpackung bzw. einzeln dem Bestrahlungsprozeß zu unterziehen.

Zur Erhöhung der Betriebssicherheit der erfindungsgemäßen Anlage 1 kann im Bereich des Eintritts des Fördersystems 3 in den Prozeßraum 27 eine Schutzeinrichtung 54 vorgesehen sein, welche beispielsweise aus einer Trennwand, einer entsprechenden Gitteranordnung oder dgl. mit einer darin angebrachten Tür 55 bestehen kann. Somit ist ein unkontrollierter Zugang zum Prozeßraum 27 in einem hohen Grad verwehrt. Zusätzlich können weitere Maßnahmen gesetzt werden, die einen derartigen unkontrollierten Zugang verhindern, beispielsweise können elektronische Verriegelungen der Schutzeinrichtung bzw. einer darin angebrachten Tür 55 den Zugang zum Prozeßraum 27 verhindern, wenn Elektronen aus der Beschleunigereinheit austreten. An der Tür 55 kann bevorzugt ein Not-Aus-Schalter 56 angebracht sein. Denkbar ist auch, daß in der Anlage 2 an beliebigen Punkten weitere Not-Aus-Schalter 56 angebracht werden.

Es ist jedoch auch möglich, die Steuerung der Beschleunigereinheit so auszubilden, daß über einen geeigneten Sensor, beispielsweise einen elektrischen Kontakt, einen optischen Sensor oder dgl., im Rahmen der Tür 55 der Behandlungsvorgang bei Öffnen der Tür 55 automatisch unterbrochen wird und somit eine potentielle Gefährdung von Personen durch austretende Elektronen beinahe zu 100 % auszuschließen ist. Als zusätzliche Sicherheitsmaßnahme kann zumindest entlang einer Teilstrecke des Fördersystems 3 beispielsweise ein Seilzugschalter 57 vorgesehen werden. Dadurch kann es Personen ermöglicht werden, welche sich unbeabsichtigt bei Aktivierung der Beschleunigereinheit im Prozeßraum 27 befinden, den Bestrahlungsprozeß zu unterbrechen bzw. bei entsprechender Auslegung einer Regel- und Steuereinrichtung 36 für die Beschleunigereinheit bei Ertönen eines optionalen akustischen Warnsignals der Regel und Steuereinrichtung 36 über den Seilzugschalter 57 mitzuteilen, daß der Linearbeschleuniger 5 in den stand-by-Betrieb zu schalten ist. Weiters ist es möglich, elektrische Kontakte, beispielsweise in einer Trittmatte hinter der Tür 55 bzw. im labyrinthartigen Zugang, anzubringen, um auf diese Weise feststellen zu können, ob Personen den Prozeßraum 27 betreten, während die Beschleunigereinheit in Betrieb ist. Dies setzt natürlich voraus, daß die Abmessungen der Trittmatte so gewählt werden, daß ein Übersteigen letzterer nicht möglich ist.

Selbstverständlich können aber auch an bestimmten Stellen in der Anlage 2, beispielsweise in der Nähe der Tür 55, Lichtschrankenanlagen angebracht sein, die den Zugang zum Prozeßraum 27 überwachen.

Eine Erhöhung der Sicherheit der Anlage 2 kann z.B. auch durch Anordnung eines Strahlunterbrechers im Strahlengang der Beschleunigereinheit erreicht werden, mit dem ein unbeabsichtigtes Austreten von Elektronen ebenfalls verhindert werden kann.

Weitere übliche Sensoren und Warneinrichtungen, wie beispielsweise Bewegungssensoren, Drehspiegelleuchten, Hupen etc. können selbstverständlich ebenfalls an jedem beliebigen Punkt der Anlag 2 angebracht sein.

Als besonders vorteilhaft erweist sich auch, wenn, wie bereits erwähnt, an neuralgischen Punkten der Anlage 2 Überwachungskameras 23 angebracht sind, mit welchen beispielsweise der Prozeßraum 27 und der Bestrahlungsraum 1 sowie die einzelnen Teile des labyrinthartigen Zuganges sowie des Fördersystems 3 überwacht werden können.

In der Nähe des Aufgabepuffers 24 kann ein Bedienpult 58 mit einem Not-Aus-Schalter 56 plaziert sein. Ein derartiges Bedienpult 58 kann sich auch im Bereich des Expreßaufgabeplatzes an der Position 39 befinden, um so die Expreßaufgabe von Gütern 4 zu ermöglichen.

Die Güter 4 auf den Transportelementen 32 können von dem in Bereichen angetriebenen Aufgabepuffer 24 an zumindest einer Markierungseinrichtung 59, beispielsweise einem Etikettenspender, einer Vorrichtung zum Anbringen von Mikrochips, einem Tintenstrahldrucker oder dgl., vorbeigeführt und dabei gleichzeitig gekennzeichnet werden. Diese Markierungseinrichtung 59 kann an jeder geeigneten, beliebigen Stelle entlang des Aufgabepuffers 24 angeordnet sein. Der Markierungseinrichtung 59 kann eine Anpreßeinrichtung, beispielsweise eine Rolle, eine Bürste oder dgl., für z.B. Etiketten zugeordnet sein. Es ist auch möglich, anstelle der Güter 4 die Transportelemente 32 bzw. sowohl die Güter 4 als auch die Transportelemente 32 zu kennzeichnen. Zur automatischen Erfassung der Güter 4 bzw. der Transportelemente 32 und zur bevorzugten Verarbeitung der erfaßten Daten in einer EDV-Anlage kann am Aufgabepuffer 24 ein Scanner 60, beispielsweise ein Lesegerät, angebracht sein. Ein derartiger Scanner 60 kann z.B. ebenfalls an dem Puffer 31 situiert sein, um so eventuell den Behandlungsgrad der Güter 4 bzw. das Vorhandensein von markierten Gütern 4 zu erkennen.

Werden als Gutmarkierung Mikrochips verwendet, so ist es beispielsweise auch möglich, diese mit einem Sender, z.B. einem IR-Sender, auszustatten, von dem aus gutspezifische Daten einer Empfangseinheit, welche vorzugsweise im Bereich des Fördersystems 3 angebracht ist, übermittelt werden können.

Die Übertragung sämtlicher ermittelter Daten, insbesondere der sensorisch ermittelten Daten, kann mit Vorteil mit sogenannten Lichtleitern erfolgen, wodurch eine Störung durch die Beeinflussung durch die Beschleunigereinheit bei der Datenübertragung vermindert werden kann.

Vorzugsweise wird die im Prozeß entwickelte Abwärme über dem Stand der Technik entsprechende Wärmetauscher zurückgewonnen und dem Prozeß wieder zugeführt.

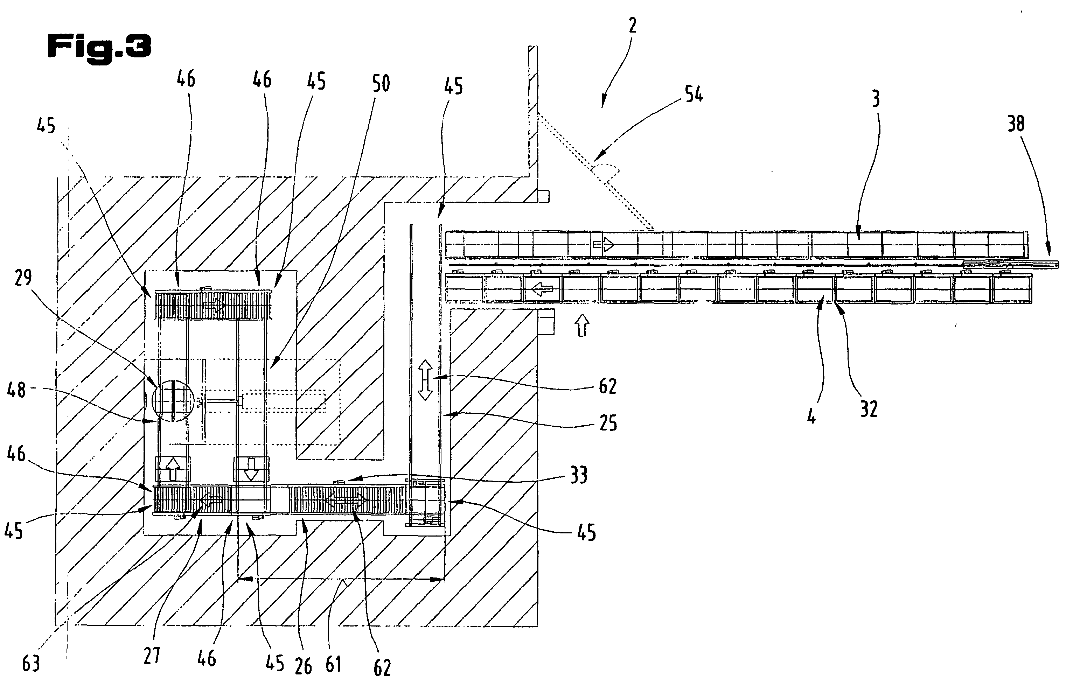

Die Fig. 3 bis 6 zeigen weitere Ausführungsvarianten für Fördersysteme 3 für eine erfindungsgemäße Anlage 2 in vereinfachter, schematischer Darstellung und Draufsicht.

So zeigt Fig. 3 eine Möglichkeit auf, wie der in Fig. 2 dargestellte Auslagerungspuffer 30 ersetzt werden kann. Es ist z.B. möglich, den Zuführpuffer 26 als Stetigförderer, beispielsweise als Rollenbahn, als Kettenförderer oder dgl., auszubilden. Dieser Stetigförderer ist in einer Art mit Antriebsvorrichtungen 33 versehen, damit er über eine Länge 61 gemäß Doppelpfeil 62 in beiden Richtungen betrieben werden kann. Dadurch wird erreicht, daß es möglich ist, die bestrahlten Güter 4 auf den Transportelementen 32 vom Prozeßabführförderer 50 nicht mehr einem wie in Fig. 2 dargestellten Auslagerungspuffer 30 zu übergeben, sondern diese erneut dem Zuführpuffer 26 zu übergeben und mit diesem aus dem Prozeßraum 27 unter Mitwirkung des Querförderers 25, welcher ebenfalls wieder gemäß dem Doppelpfeil 62 in beiden Richtungen betrieben werden kann, zu entfernen. Um das Umsetzen der Transportelemente 32 in den speziellen Bereichen des Fördersystems 3 speziell in den Ecken zu ermöglichen, können an sämtlichen Übergabebereichen 45 wiederum Senk- und Hebevorrichtungen 46 vorhanden sein. Des weiteren ist es möglich, einzelne Elemente des Fördersystems 3 bzw. die diesen zugeordneten Positionserkennungsvorrichtungen 34, welche in Fig. 2 dargestellt sind, bzw. weitere ebenfalls dem Fördersystem 3 der Fig. 2 zugehörigen Teile ebenfalls im Fördersystem 3 der Fig. 3 zu verwenden, auch wenn diese nicht dargestellt sind.

Fig. 3 zeigt weiters eine Möglichkeit auf, wie der Prozeßförderer 29 an einer anderen Stelle als der in Fig. 2 dargestellten im Prozeßraum 27 angebracht sein kann.

Mit dem Fördersystem 3 der Fig. 3 ist es nicht nur möglich, die Wirtschaftlichkeit der Anlage 2 zu erhöhen - es kann, wie bereits erwähnt, auf den Auslagerungspuffer 30 der Fig. 2 verzichtet werden - sondern können die Güter 4 auf den Transportelementen 32 einer mehrfachen Bestrahlung unterzogen werden, indem sie nach einem ersten Bestrahlungsvorgang den Zuführpuffer 26 nicht über die Länge 61 verlassen, sondern erneut gemäß Pfeil 63 dem Bestrahlungsprozeß über den Prozeßzuführförderer 48 zugeführt werden.

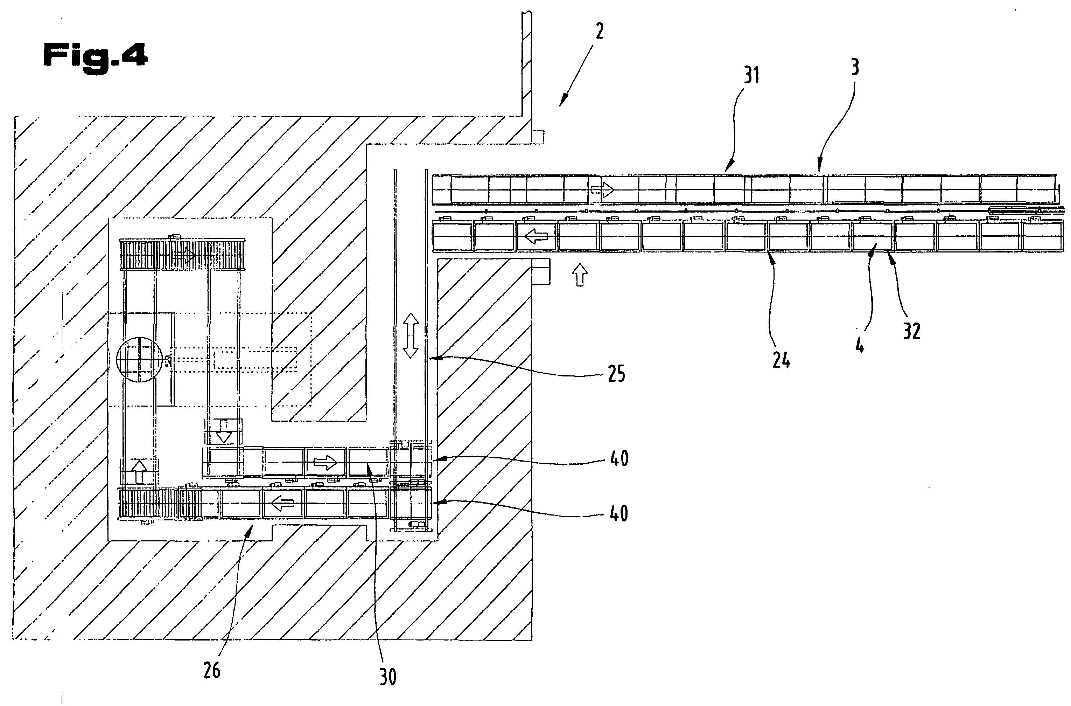

Selbstverständlich ist es möglich, sämtliche in Fig. 1 und Fig. 2 beschriebenen Teile der Anlage 2, beispielsweise die Trennvorrichtung 38, die Schutzeinrichtung 54, sowohl bei der in Fig. 3 dargestellten Anlage 2 als auch bei den in Fig. 4 bis Fig. 6 dargestellten Anlagen 2 zu verwenden. Aus diesem Grund wird in den folgenden Fig. 4 bis 6 nur auf das Fördersystem 3 eingegangen.

Fig. 4 zeigt in schematischer Darstellung eine weitere Möglichkeit auf, wie das Fördersystem 3 für eine erfindungsgemäße Anlage 2 ausgebildet sein kann. Dabei ist der Querförderer 25 bevorzugt wiederum als Querverfahrwagen 40 ausgebildet, wobei jedoch zur Erhöhung der Kapazität des Fördersystems 3 zumindest zwei Querverfahrwagen 40 verwendet werden. Bevorzugt wird dabei jeweils ein separater Querverfahrwagen 40 für den Aufgabepuffer 24, der die Güter 4 auf den Transportelementen 32 dem Zuführpuffer 26 übergibt sowie einen Querverfahrwagen 40 für den Puffer 31 eingesetzt, um so die Transportelemente 32, welche sich auf dem Auslagerungspuffer 30 befinden, unabhängig vom Querverfahrwagen 40 des Aufgabepuffers 24 transportieren zu können.

Die Bevorzugung von getrennten Querverfahrwagen 40 schließt jedoch die Möglichkeit nicht aus, die beiden Querverfahrwagen 40 miteinander zu koppeln, wobei jedoch der Abstand der beiden Querverfahrwagen 40 auf den Abstand des Aufgabepuffers 24 vom Puffer 31 bzw. des Zuführpuffers 26 vom Auslagerungspuffer 30 abgestimmt werden soll.

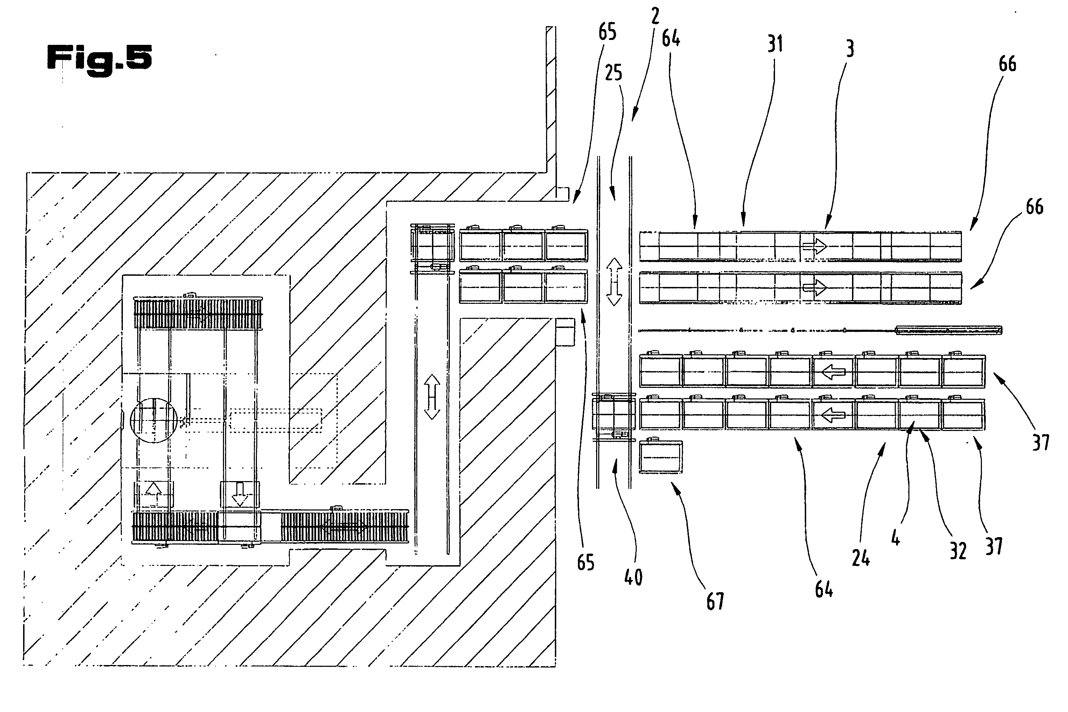

Die in Fig. 5 dargestellte Ausführungsvariante eines Fördersystems 3 für die Anlage 2 zeigt eine weitere Möglichkeit auf, mit der die Kapazität der Anlage 2 gesteigert werden kann. Dabei können sowohl der Aufgabepuffer 24 als auch der Puffer 31 bevorzugt in jeweils zwei getrennte Bereiche 64 und 65 unterteilt sein. Somit ist es möglich, im Bereich 64 einerseits den Aufgabepuffer 24 und/oder andererseits den Puffer 31 zumindest zweiteilig auszuführen, sodaß am Aufgabepuffer 24 zumindest zwei getrennte Aufgabeplätze 37 und/oder am Puffer 31 zwei getrennte Abnahmeplätze 66 vorhanden sind. Des weiteren kann im Bereich 64 des Aufgabepuffers 24 wiederum ein Schnell-Aufgabeplatz 67 vorhanden sein, um eine vorzugsweise Behandlung von einzelnen Gütern 4 auf Transportelementen 32 zu ermöglichen.

Um die Güter 4 auf den Transportelementen 32 einerseits vom Bereich 64 des Aufgabepuffers 24 dem diesem zugeordneten Bereich 65 bzw. vom Bereich 65 dem Bereich 64 des Puffers 31 zum Abtransport zu übergeben, kann entweder ein Stetigförderer oder, wie in Fig. 5 dargestellt, ein zusätzlicher Querförderer 25, beispielsweise ein Querverfahrwagen 40, verwendet werden. Selbstverständlich ist es hierbei wiederum möglich, mehrere Querverfahrwagen 40 einzusetzen.

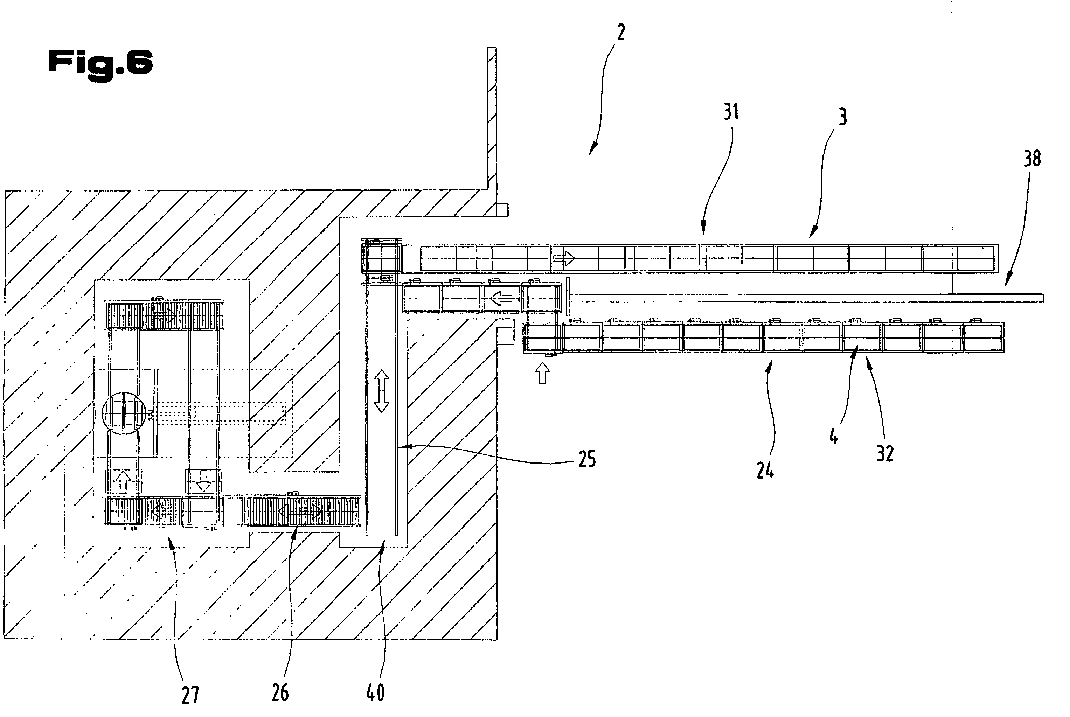

Das in Fig. 6 schematisch dargestellte Fördersystem 3 für die Anlage 2 zeigt auf, wie auf einfache Art und Weise die Möglichkeit geschaffen werden kann, zumindest Teile des unreinen Aufgabepuffers 24 vom reinen Puffer 31 zu trennen und dabei gleichzeitig eine gute Erreichbarkeit des Aufgabepuffers 24 und des Puffers 31 von mehreren Seiten, beispielsweise für Wartungsarbeiten, zu ermöglichen. Die Trennung erfolgt, wie schon ausgeführt, bevorzugt über eine Trennvorrichtung 38.

Eine derartige Ausführungsvariante des Fördersystems 3 kann bevorzugt immer dann eingesetzt werden, wenn beispielsweise für den Querförderer 25 ein gekoppelter Querverfahrwagen 40 (in Fig. 6 aufgrund der gewählten Ausführung der Teile des Fördersystems 3 im Prozeßraum 27 getrennt dargestellt) verwendet wird, um so die Zufuhr von Gütern 4 auf Transportelementen 32 zum Zuführpuffer 26 bzw. den Abtransport der Güter 4 vom Auslagerungspuffer 30 (in Fig. 6 nicht dargestellt) bei geringem Flächenbedarf des Prozeßraumes 27 zu ermöglichen.

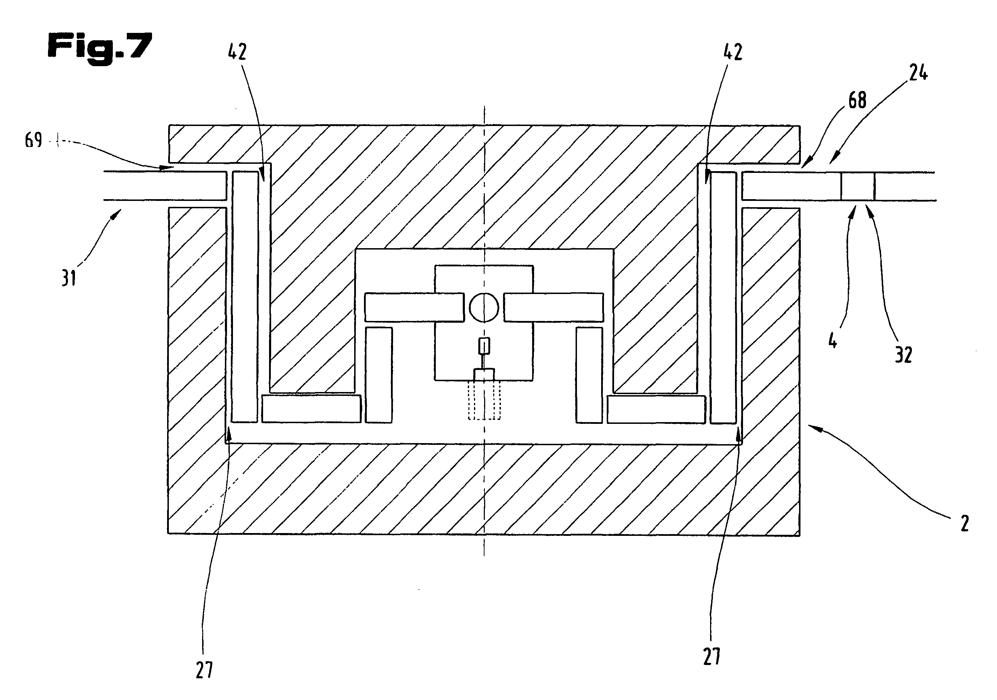

Durch die Ausführungsvariante einer erfindungsgemäßen Anlage 2, welche in Fig. 7 vereinfacht und schematisch als Draufsicht dargestellt ist (zur Verbesserung der Übersichtlichkeit wurde wiederum auf die Darstellung jener Teile, welche bevorzugt unterirdisch angeordnet sind, verzichtet), ist es auf einfache Art und Weise möglich, die erfindungsgemäße Anlage 2 beispielsweise in eine Fertigungsstraße einzubinden.

Die Zufuhr von Gütern 4 auf Transportelementen 32 erfolgt dabei wiederum über den Aufgabepuffer 24, welcher in einem Eingangsbereich 68 des Labyrinths 42 die Transportetemente 32 mit den Gütern 4 dem weiteren Fördersystemteil des Fördersystems 3 übergibt. Im Gegensatz zu den bisher beschriebenen Ausführungsvarianten befindet sich aber ein Ausgangsbereich 69 eines zweiten Labyrinths an einer anderen Stelle der Anlage 2 als das erste Labyrinth 42 des Eingangsbereiches 68. Bevorzugt sind der Eingangsbereich 68 und der Ausgangsbereich 69 einander gegenüberliegend in der Anlage 2 angeordnet.

Mit einer derartigen Anordnung des Fördersystem 3 kann zudem eine noch bessere Trennung des unreinen Aufgabepuffers 24 vom reinen Puffer 31 erreicht werden.

Im übrigen können die einzelnen Fördersystemteile aus den bereits genannten Teilen gebildet sein bzw. können diesen die entsprechenden und bereits angeführten erforderlichen Systemteile zugeordnet sein.

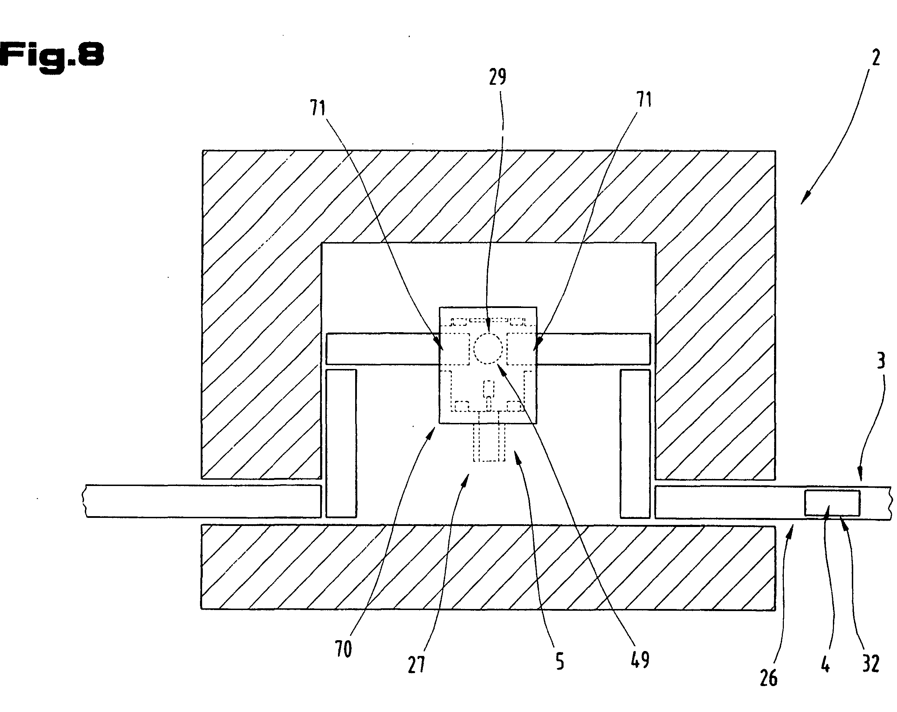

Fig. 8 zeigt eine weitere Ausführungsvariante einer erfindungsgemäßen Anlage 2 in vereinfachter, schematischer Darstellung und Draufsicht. Aufgrund des Umstandes, daß die Beschleunigereinheit, insbesondere der Linearbeschleuniger 5, bevorzugt unterirdisch angeordnet ist und dadurch das umgebende Erdreich zur Abschirmung ausgenutzt werden kann, ist es möglich, beispielsweise bei Anlagen 2 mit geringer Leistung auf die Anordnung eines labyrinthartigen Zuganges zum Prozeßraum 27 zu verzichten. Anstelle eines Labyrinths 42 kann, wie in Fig. 8 dargestellt, der Bereich, in dem sich der Prozeßförderer 29, z.B. der Dreh-Hubtisch 49, befindet, durch zusätzliche bauliche Maßnahmen gesichert werden. Beispielsweise kann eine Variante eines zusätzlichen Gehäuses 70 über dem Prozeßförderer 29 gewählt werden. Als Materialien für das Gehäuse 70 können sämtliche dem Verwendungszweck entsprechende Werkstoffe verwendet werden, beispielsweise Stahlbeton mit oder ohne zusätzlicher Metallauskleidung. Bevorzugt werden solche Metalle eingesetzt, welche einen hohen Einfangquerschnitt für Elektronen aufweisen.

Ist das Fördersystem 3 wie in Fig. 8 dargestellt ausgebildet, beispielsweise für die Einbindung der Anlage 2 in eine Förderstraße, so können am Gehäuse 70 an jeweils bevorzugt gegenüberliegenden Seiten Gehäusedurchbrüche 71 vorhanden sein, mit denen es möglich wird, Güter 4 auf Transportelementen 32 dem Prozeßförderer 29 zuzuführen.

Dabei sind die Abmessungen für die Gehäusedurchbrüche 71 bevorzugt auf die maximalen Ausmessungen der Transportelemente 32 mit Gütern 4 abgestimmt.

Selbstverständlich ist es auch möglich, anders wie in Fig. 8 dargestellt, den Zugang und den Ausgang zum Prozeßraum 27 auf der gleichen Seite der Anlage 2 anzuordnen bzw. ist es möglich, daß bei geeigneter Konstruktion des Fördersystems 3 nur ein Zuführpuffer 26 vorhanden ist, der auch den Abtransport der Transportelemente 32 mit den Gütern 4 übernimmt.

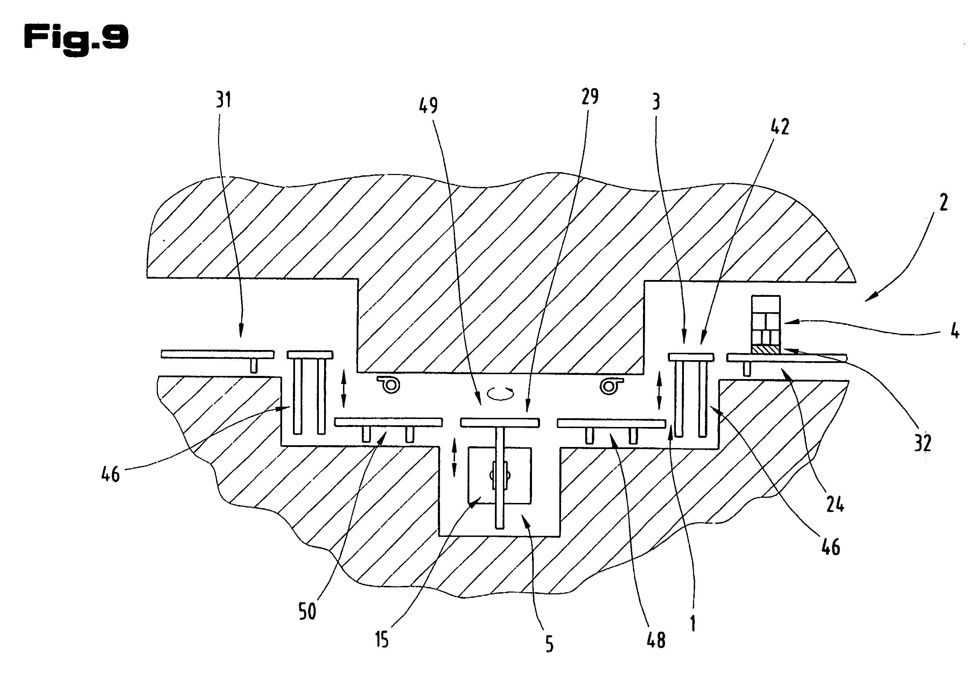

Fig. 9 zeigt als Ausführungsvariante einer erfindungsgemäßen Anlage 2 im Querschnitt eine weitere Möglichkeit auf, wie die Anlage 2 vorzugsweise in eine Fertigungsstraße eingebunden werden kann. Anstelle eines horizontalen Labyrinths 42, wie in den vorangehenden Figuren zum Teil beschrieben, findet bei der Ausführungsvariante der Fig. 9 ein senkrechtes Labyrinth 42 Verwendung. Dadurch wird eine kostengünstige und platzsparende Variante der Anlage 2 geschaffen, wie es insbesondere auch im Einsatz in Fertigungsstraßen wünschenswert ist. Güter 4, welche sich auf Transportelementen 32 befinden, werden dabei vom Aufgabepuffer 24 an eine erste Hebevorrichtung 46 übergeben, welche die Güter 4 in den Bestrahlungsraum 1 verbringt. Dort werden sie von dem Prozeßzuförderer 48 übernommen und in der Folge dem Prozeßförderer 29, beispielsweise dem Dreh-Hubtisch 49, übergeben. Auf diesem werden die Güter 4, wie bereits beschreiben, durch beispielsweise kombinierte Vertikal- und Drehbewegung an der Elektronenaustrittsvorrichtung 15 des Linearbeschleunigers 5 zur Bestrahlung vorbeibewegt. Nach erfolgter Bestrahlung, welche sowohl während des Absenkens als auch während des Anhebens der Güter 4 erfolgen kann, werden diese über den Prozeßabführförderer 50 sowie einer dritten Hebevorrichtung 46 und dem Puffer 31 dem Bestrahlungsprozeß entzogen.

Die Lenkung des Bestrahlungsprozesses kann auch hier wiederum mit Hilfe von bereits beschriebenen und in Fig. 9 nicht dargestellten Positionserkennungsvorrichtungen 34, welche ihrerseits Antriebsvorrichtungen 33 steuern, erfolgen.

Selbstverständlich ist es auch bei dieser Variante möglich, anstelle von zwei getrennten Labyrinthen 42 nur eines sowohl für die Zufuhr als auch den Abtransport der Güter 4 zum bzw. aus dem Bestrahlungsraum 1 zu verwenden. Es können auch einzelne Teile des Fördersystems 3 sowohl für die Zufuhr als auch für den Abtransport der Güter 4 verwendet werden bzw. ist es möglich, zumindest zwei getrennte Förderlinien nebeneinander zu führen.

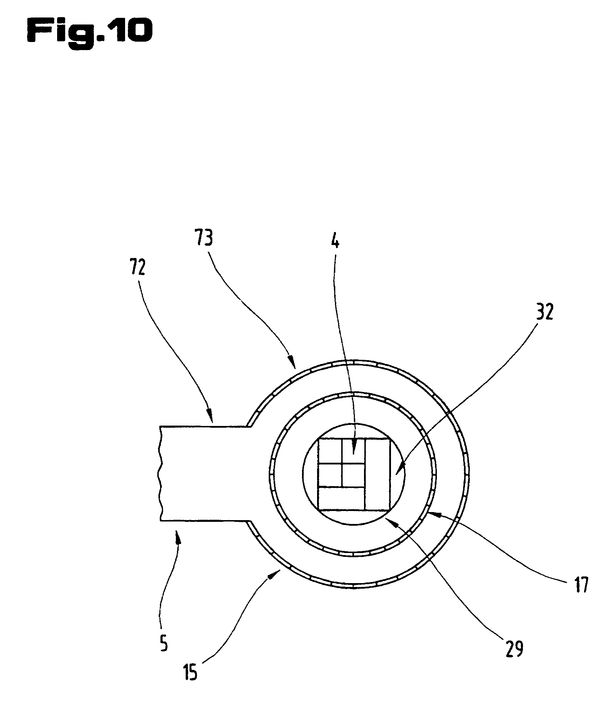

In Fig. 10 ist schließlich eine Ausführungsvariante für eine Elektronenaustrittsvorrichtung 15 in vereinfachter und schematischer Darstellung gezeigt. Die Elektronenaustrittsvorrichtung 15 eines Elektronenbeschleunigers 72, insbesondere des Linearbeschleunigers 5, ist dabei, wie die Draufsicht der Fig. 10 zeigt, in Form eines Kreises angeordnet. Zur Ablenkung der Elektronen können beispielsweise entlang des äußeren Umfanges der Elektronenaustrittsvorrichtung 15 Ablenkeinrichtungen 73, beispielsweise elektrische Spulen, welche aufgrund der Gesetze des Elektromagnetismus von stromdurchflossenen Leitern ein Magnetfeld aufbauen, angeordnet sein. Weiters können am inneren Umfang der Elektronenaustrittsvorrichtung 15 (in Fig. 10 nicht dargestellt) sogenannte Beam-Stops 17 vorhanden sein.

Bei einer derartigen Ausbildung einer Elektronenaustrittsvorrichtung 15 ist es möglich, auf eine Drehbewegung des Prozeßförderers 29 zu verzichten, sodaß die Güter 4 auf Transportelementen 32 einzig während einer Vertikalbewegung bestrahlt werden. Dies kann sowohl während des Absenkens als auch des Anhebens des Prozeßförderers 29 erfolgen. Selbstverständlich besteht aber auch die Möglichkeit, eine kombinierte Vertikal- und Drehbewegung des Prozeßförderers 29 während des Bestrahlungsvorganges auszuführen.

Als Verfahren zur Bestrahlung von Gütern 4 auf Transportelementen 32 hat sich die im folgenden beschriebene Vorgangsweise, die jedoch nicht zwingend ist und gegebenenfalls adaptiert werden kann, als vorteilhaft erwiesen.

Das zu bestrahlende Gut 4 wird über den unreinen Aufgabepuffer 24, insbesondere den Aufgabeplatz 37, dem Fördersystem 3 zugeführt. Dies kann beispielsweise händisch durch Herabnehmen der Güter 4 von Transportelementen 32, beispielsweise Paletten und Umschlichten der Güter 4 auf Transportelemente 32 auf dem Aufgabepuffer 24 erfolgen.

Bevorzugt werden aber von einem Fertigungsprozeß bereitgestellte, zum Transport mit beispielsweise LKW's bestimmte, fertig mit Gütern 4 beladene Transportelemente 32 verwendet. Diese können auch bereits mit einer Schrumpffolie umwickelt sein.

Zur individuellen Kennzeichnung der Güter 4 und/oder der Transportelemente 32 werden diese an einer Markierungseinrichtung 59, beispielsweise einem Etikettenspender, einem Tintenstrahldrucker oder dgl., vorbeigeführt. Die derart angebrachte Markierung, welche z.B. aus einem Strich-Code bestehen kann, wird von einem nachfolgenden Scanner 60 erfaßt und diese Daten einer Regel- und/oder Steuereinrichtung 36, beispielsweise einer EDV-Anlage zugeführt.

Daraufhin werden die Güter 4 über die einzelnen Teile des Fördersystems 3 in den Prozeßraum 27 und in der Folge den Bestrahlungsraum 1 verbracht. Für die im Gut 4 zu deponierende Dosis an Strahlenenergie ist es unter anderem wichtig, daß die Vortriebsgeschwindigkeit des Prozeßförderers 29 auf die Taktfrequenz der Elektronenpulse der Elektronenbeschleunigereinheit abgestimmt wird. Da für die Beschleunigung der Elektronen gepulste elektromagnetische Wellen mit vordefinierter Frequenz, welche als stehende Wellen in Hohlraumresonatoren 7 vorliegen, die von einer gepulsten Mikrowelle, erzeugt von einem Oszillator 8, angeregt und von einem Mikrowellenverstärker 9, beispielsweise einem Klystron oder dgl., verstärkt werden, hat es sich als vorteilhaft erwiesen, wenn die Pulse der Elektronenstrahlung um mindestens 30 %, vorzugsweise 50 %, überlappen. Auf diese Weise ist es möglich, im Gut 4 eine homogene Dosisverteilung zu erreichen.

Vorteilhaft ist eine Beschleunigeranlage, die eine Strahlenenergie im Bereich von 5 MeV bis 30 MeV, bevorzugt 10 MeV, und eine mittlere Beamleistung im Bereich von 5 kW bis 40 kW, vorzugsweise 10 kW bis 30 kW, insbesondere 20 kW, zur Verfügung stellt.

Die Vortriebsgeschwindigkeit des Prozeßförderers 29, insbesondere des Dreh-Hubtisches 49, sollte zwischen 1 Umdrehung/s und 1 Umdrehung/5 s, bevorzugt 1 Umdrehung/3 s, betragen.

Die Bestrahlung des Gutes 4 erfolgt vorzugsweise parallel zu einer Teilbewegungsrichtung des Gutes 4 auf dem Prozeßförderer 29, kann aber auch von mehreren Seiten durchgeführt werden, vor allem dann, wenn mehrere Elektronenbeschleuniger eingesetzt werden. Im letzteren Fall kann das Gut 4 alternierend oder simultan von mehreren Seiten bestrahlt werden.

Nach erfolgter Bestrahlung wird das Gut 4 über den labyrinthartigen Zugang aus dem Bestrahlungsraum 1 bzw. dem Prozeßraum 27 transportiert und an einem Erkennungssystem, beispielsweise einem Scanner 60, insbesondere einem Lesegerät, vorbeigeführt. Die damit erfaßten Daten können ebenfalls einer EDV-Anlage zur Verfügung gestellt werden, welche auf diese Weise den Bestrahlungsgrad des Gutes 4 feststellen kann. Ist es erforderlich, daß das Gut 4 mehrmals bestrahlt wird, werden diese Daten einzelnen Antriebsvorrichtungen 33 zur Verfügung gestellt, die beispielsweise in Abhängigkeit vom Querförderer 25, Güter 4, welche sich auf dem Aufgabepuffer 24 befinden, vom Weitertransport in den Prozeßraum 27 abhalten. Dadurch wird es möglich, über den Querförderer 25 bereits bestrahlte Güter 4 erneut dem Bestrahlungsprozeß zuzuführen. Die Güter 4, die sich auf dem Aufgabepuffer 24 befinden, können von einem ersten Gutstop, der beispielsweise am Übergabebereich Aufgabepuffer 24/Querförderer 25 plaziert sein kann, solange aufgehalten und gepuffert werden, bis das erste fertig bestrahlte Gut 4 vom Querförderer 25 dem Puffer 31 übergeben worden ist. Die Mehrfachbestrahlung wird wiederum über den Scanner 60 erfaßt.

Selbstverständlich ist es möglich, bei entsprechender Ausgestaltung des Fördersystems 3, wie beispielsweise in Fig. 3 beschrieben, die Güter 4 auf den Transportelementen 32 an einer anderen Stelle des Transportsystems zu puffern.

Es ist aber auch möglich, gerade durch die spezielle Ausbildung des Prozeßförderers 29 als Dreh-Hubtisch 49 die Mehrfachbestrahlung der Güter 4 durch den Bewegungsablauf des Prozeßförderers 29 zu erreichen, sodaß eine zusätzliche Pufferung der Güter 4 an diversen Stellen des Fördersystems 3 nicht mehr notwendig ist.

Mit einer derartigen Steuerung wird auf vorteilhafte Weise eine sehr gute Auslastung der zur Verfügung stehenden Ressourcen erreicht. Fakultativ zum ersten Gutstop können über die Länge des Fördersystems 3 weitere Gutstops angebracht sein. Vorzugsweise sind diese vor jeder Richtungsänderung des Transportsystems plaziert. Dadurch wird ein Puffer an Gütern 4 aufgebaut, sodaß in der Folge eine geringe Auslastung des Prozeßförderers 29 weitestgehend verhindert wird. Die Güter 4 werden von den jeweiligen Gutstops in definierten Intervallen freigegeben, deren Zeitspanne sich nach der einzubringenden Strahlendosis richtet.

In besonderen Fällen kann durch wiederholte Änderung der Bewegungsrichtung der Güter 4, d.h. von Teilen des Fördersystems 3, insbesondere des Prozeßförderers 29, diese zur Erreichung der notwendigen Strahlendosis im Gut 4 mehrmals am Elektronenstrahl vorbeigeführt werden.

Üblicherweise erfolgt der Zu- und Abtransport der Güter 4 zu und aus dem Prozeßraum 27 auf einer Seite des Prozeßraumes 27. Es ist jedoch auch möglich, die Zufuhr und den Abtransport der Güter 4 zu und aus dem Prozeßraum 27 auf unterschiedlichen Seiten des Prozeßraumes 27 vorzunehmen.

Zur Ermittlung der erforderlichen Dosis können vorab an einzelnen Gütern 4 Dosimeter, beispielsweise radiochrome Filmdosimeter, an signifikanten Stellen angebracht und diese Güter 4 dem Bestrahlungsprozeß ausgesetzt werden. Derartige signifikante Stellen sind beispielsweise jene Stellen, die sich durch erhöhte bzw. zu geringe Dosisübertragung auszeichnen. Dies ist vor allem im Inneren, aber auch an den Eckpunkten der Güter 4 zu erwarten, besonders dann, wenn die Güter 4 in einer Verpackung, beispielsweise ihrer Originalverpackung, bestrahlt werden, sodaß die zu verwendenden Dosimeter bevorzugt an diesen Stellen einzusetzen sind.

Radiochrome Filmdosimeter zeichnen sich dadurch aus, daß sie ihre Farbe in Abhängigkeit von der Bestrahlungsdauer bzw. der verabreichten Dosis ändern. Diese Farbänderung kann in der Folge über ein Spektralphotometer durch Messung der Abschwächung der Intensität eines hindurchtretenden Lichtstrahles bzw. in Reflexion gemessen werden. Aus den dabei ermittelten Werten läßt sich die exakte Vortriebsgeschwindigkeit des Prozeßförderers 29 bei konstanter Beam-Leistung errechnen. Für die Erstellung der Kalibrierungskurve zur Auswertung der Filme haben sich Alanin Transferdosimeter als vorteilhaft erwiesen. Selbstverständlich ist es aber auch möglich, sämtliche andere Methoden zur Kalibrierung zu verwenden.

Zusätzlich zu radiochromen Filmdosimetern können auch zur Erhöhung der Sicherheit weitere Dosimeter, vorzugsweise kalorimetrische Dosimeter eingesetzt werden.

Zur weiteren Sicherheitssteigerung können während des Produktionsprozesses in definierten Abständen weitere Filmdosimeter an den Gütern 4 angebracht werden, vorzugsweise Dosimeter mit derselben Markierung oder Kennung, wie sie für die Güter 4 verwendet werden. Auf diese Weise ist eine spätere unbeabsichtigte Verwechslung der einzelnen Filmdosimeter zu vermeiden. Diese mit den zusätzlichen Dosimetern versehenen Güter 4 können von einer Regel- und/oder Steuereinrichtung 36 über beispielsweise den Scanner 60 erkannt werden.

Zur weiteren Nachbehandlung sowie zur Kontrolle der Stabilität des Prozesses können die aus den Dosimetern gewonnen Daten einer Datenverarbeitungsanlage zugeführt und von dieser archiviert werden.

Es ist möglich, in der Anlage 2 das Gut 4 mit dem Fördersystem 3 mit einer Geschwindigkeit an der Elektronenaustrittsvorrichtung 15 der Beschleunigereinheit, insbesondere dem Scan-Horn, vorbeibewegt wird. die sich nach der zu verabreichenden Dosis richtet. Dadurch ist mit Vorteil eine universelle Anwendbarkeit des Verfahrens für Güter unterschiedlichster Art möglich.

Weiters ist es möglich, daß mehrere Güter bzw. mehrere mit Gütern versehene Transportbehälter, beispielsweise Karton-, Kunststoffverpackungen oder dgl., ohne Zwischenraum an der Austrittsstelle des Elektronenstrahls aus der Beschleunigereinheit vorbeitransportiert werden. Dadurch kann eine Steigerung des Auslastungsgrades der Beschleunigungereinheit erreicht werden.

Der Antrieb einzelner Teile des Fördersystems kann mit einem Servomotor erfolgen, wodurch ein reibungsloser Betrieb des Fördersystems 3 gewährleistet und die jeweils benötigten Vortriebsgeschwindigkeiten fein auf die Eigenheiten des zu bestrahlenden Gutes 4 abgestimmt werden können.

Mit einer erfindungsgemäßen Anlage 2 ist es vorteilhafterweise möglich, Güter 4 unterschiedlicher Art zu behandeln bzw. deren Eigenschaften in Teilbereichen zu verändern. So können beispielsweise Produkte unterschiedlichster Art sterilisiert werden. Dazu gehören unter anderem OP-Equipment, OP-Kleidung, Verbandsstoffe, OP-Abfall, Pharmarohstoffe, Pharmaverpackungen, Behältnisse aus Kunststoff und/oder Glas, Versuchsbehältnisse bzw. Versuchsequipment für den Biotechnologiebereich, die Sterilisation von Flüssigkeiten, Recyclingmaterialien und Müll im Bereich der Umwelttechnologie, die Entkeimung von Kunststoffen, die Sterilisation und Keimreduktion in Gewürzen, Rohstoffen, Produkten, Getränken, Verschlüssen sowie die Sterilisation von Verpackungen, Behältern oder Gefäßen für die Verpackungstechnologie.

Neben der Sterilisation gibt es aber eine Fülle von Einsatzmöglichkeiten für eine Anlage 2 der erfindungsgemäßen Art. Dazu gehört unter anderem die Veredelung von Oberflächen, beispielsweise durch Härten, die Härtung bzw. Vernetzung von Kunststoffen, die Aushärtung bzw. Vernetzung von Lacken. Bei Auswahl eines geeigneten Transportmittels, beispielsweise in Form von Flaschen, können selbst Flüssigkeiten, z.B. Bier, Wasser oder dgl., und Gase auf diese Weise bestrahlt werden. Weiters können Schütt- und Stückgüter unterschiedlichster Art mit der erfindungsgemäßen Anlage 2 bestrahlt werden.

Der Ordnung halber sei abschließend darauf hingewiesen, daß zum besseren Verständnis des Aufbaus der Ausführungsvarianten für eine erfindungsgemäße Anlage 2 diese bzw. deren Bestandteile teilweise unmaßstäblich und/oder vergrößert und/oder verkleinert dargestellt wurden.

### Bezugszeichenaufstellung

- 1: Bestrahlungsraum
- 2: Anlage
- 3: Fördersystem
- 4: Gut
- 5: Linearbeschleuniger

- 6: Glühkathode
- 7: Hohlraumresonator
- 8: Oszillator
- 9: Mikrowellenverstärker
- 10: Hochspannungsmodulator

- 11: Energiequelle
- 12: Quadrupol
- 13: Shutter
- 14: Ablenkmagnet
- 15: Elektronenaustrittsvorrichtung

- 16: Wand
- 17: Beam-Stop
- 18: Entlüftungsvorrichtung
- 19: Raum
- 20: Wand

- 21: Wand
- 22: Durchbruch
- 23: Überwachungskamera
- 24: Aufgabepuffer
- 25: Querförderer

- 26: Zuführpuffer
- 27: Prozeßraum
- 28: Stetigförderer
- 29: Prozeßförderer
- 30: Auslagerungspuffer

- 31: Puffer
- 32: Transportelement
- 33: Antriebsvorrichtung
- 34: Positionserkennungsvorrichtung
- 35: Lichtschranke

- 36: Steuereinrichtung
- 37: Aufgabeplatz
- 38: Trennvorrichtung
- 39: Position
- 40: Querverfahrwagen

- 41: Schiene
- 42: Labyrinth
- 43: Strecke
- 44: Kettenförderer
- 45: Übergabebereich

- 46: Hebevorrichtung
- 47: Förderer
- 48: Prozeßzuführförderer
- 49: Dreh-Hubtisch
- 50: Prozeßabführförderer

- 51: Pfeil
- 52: Pfeil
- 53: Staplerabnahmeplatz
- 54: Schutzeinrichtung
- 55: Tür

- 56: Not-Aus-Schalter
- 57: Seilzugschalter
- 58: Bedienpult
- 59: Markierungseinrichtung
- 60: Scanner

- 61: Länge
- 62: Doppelpfeil
- 63: Pfeil
- 64: Bereich
- 65: Bereich

- 66: Abnahmeplatz
- 67: Schnell-Aufgabeplatz
- 68: Eingangsbereich
- 69: Ausgangsbereich
- 70: Gehäuse

- 71: Gehäusedurchbruch
- 72: Elektronenbeschleuniger
- 73: Ablenkeinrichtung

## Patentansprüche

1. Verfahren zum Bestrahlen eines Gutes, insbesondere eines Materials, eines Abfalls, eines Bauteils, eines Lebensmittels, einer Flüssigkeit, eines Gases, bei dem das Gut, gegebenenfalls in einer Verpackung und/oder auf einem Transportelement, mit einem einen Prozeßförderer umfassenden Fördersystem durch einen Strahl bewegter Elektronen aus zumindest einem Elektronenbeschleuniger in einer Bestahlungskammer bewegt wird, wobei die zur Bestrahlung benötigten, aus einer Glühkathode austretenden Elektronen fokusiert und in einer Beschleunigereinheit mit Wellen einer bestimmten, vordefinierbaren Frequenz gepulst werden und mit einer bestimmten Frequenz aus der Elektronenaustrittsvorrichtung austreten und auf das zu bestrahlende Gut gelenkt werden, **dadurch gekennzeichnet, daß** der Prozeßförderer eine aus einer Vertikal- und Drehbewegung zusammengesetzte Bewegung während der Bestrahlung ausführt.

2. Verfahren nach Anspruch 1, **dadurch gekennzeichnet, daß** die Vortriebsgeschwindigkeit zumindest eines Teils des Fördersystems, die Scanhöhe und die Zahl der Pulse sowie die Pulsdauer so aufeinander abgestimmt werden, daß sich die Pulse der Elektronenstrahlung um mindestens 30 %, vorzugsweise 50 %, überlappen.

3. Verfahren nach Anspruch 1 und/oder 2, **dadurch gekennzeichnet, daß** die Vortriebsgeschwindigkeit des Fördersystems, vorzugsweise nach dem Aufbau von Puffern, auf die Verweildauer des Gutes am Prozeßförderer abgestimmt ist.

4. Verfahren nach einem der vorhergehenden Ansprüche, **dadurch gekennzeichnet, daß** das Gut alternierend oder gleichzeitig von mehreren Seiten bestrahlt wird, vorzugsweise in auf das Fördersystem bezogener vertikaler bzw. horizontaler Richtung.

5. Verfahren nach einem der vorhergehenden Ansprüche, **dadurch gekennzeichnet, daß** zur Messung der in das Gut eingebrachten Energiedosis radiochome Filmdosimeter verwendet werden und für die Erstellung einer Kalibrierungskurve zur Auswertung der Filme Alanin Transferdosimeter verwendet werden.

6. Verfahren nach Anspruch 5, **dadurch gekennzeichnet, daß** nach der Auswertung der Filmdosimeter durch Messung der Intensität eines das Filmdosimeter durchdringenden Lichtstrahls in Transmission die erfaßten Kennwerte einer EDV-Anlage zur Nachbearbeitung, Speicherung und Kontrolle übergeben werden.

7. Verfahren nach einem der vorhergehenden Ansprüche, **dadurch gekennzeichnet, daß** an dem Gut und/oder der Verpackung des Gutes und/oder einem Transportelement zumindest eine Markierung, beispielsweise ein Strich-Code, angebracht wird, die von einem Erkennungssystem, beispielsweise einem Scanner, insbesondere einem Lesegerät, erkannt wird.

8. Verfahren nach einem der Ansprüche 5 bis 7, **dadurch gekennzeichnet, daß** an dem Filmdosimeter zumindest eine Markierung zur nachträglichen Auswertung, Erkennung und Überprüfung des Filmdosimeters, insbesondere ein Strich-Code, angebracht wird, die von einem Erkennungssystem, beispielsweise einem Scanner, insbesondere einem Lesegerät, erkannt wird.

9. Verfahren nach Anspruch 8, **dadurch gekennzeichnet, daß** das Gut nach dem Erkennungssystem gepuffert wird.

10. Verfahren nach einem der vorhergehenden Ansprüche, **dadurch gekennzeichnet, daß** das Gut bzw. die Güter an zumindest zwei vorbestimmten Stellen des Fördersystems, insbesondere an den Stellen, an denen eine Änderung der Bewegungsrichtung stattfindet, gepuffert wird bzw. werden.

11. Verfahren nach einem der vorhergehenden Ansprüche, **dadurch gekennzeichnet, daß** das Gut bzw. die Güter auf dem Transportelement in aus dem Behandlungsgrad errechneten Intervallen einer Zuführfördereinrichtung dem Beschleunigerraum zugeführt werden.

12. Verfahren nach einem der vorhergehenden Ansprüche, **dadurch gekennzeichnet, daß** ein Behandlungsgrad des Gutes von einem Scanner, beispielsweise einem Strich-Code-Lesegerät, erfaßt wird.

13. Verfahren nach einem der vorhergehenden Ansprüche, **dadurch gekennzeichnet, daß** das Gut über eine Abnahmefördereinrichtung, bevorzugt eine Schwerkraftbahn mit flurebenem Abnahmeplatz, dem Prozeß entzogen wird.

14. Verfahren nach einem der vorhergehenden Ansprüche, **dadurch gekennzeichnet, daß** der Elektronenstrahl die Oberfläche des Gutes in Form einer geometrischen, vorzugsweise annähernd wellenartigen Kurve überstreicht, die durch die Kombination der Bewegung des Gutes mit der Bewegung des Elektronenstrahls festzulegen ist.

15. Verfahren nach einem der vorhergehenden Ansprüche, **dadurch gekennzeichnet, daß** an zumindest einem Gut in einem Vorversuch die benötigte Strahlendosis mittels an markanten Stellen des Gutes, insbesondere an jenen Stellen, an denen die Strahlenbelastung wegen des Scan-Modus gering und/oder hoch ausfällt, beispielsweise in der Mitte der Oberfläche und/oder im Inneren des Gutes, in den Ecken des Gutes, angebrachten Dosimetern ermittelt wird und die ermittelte benötigte Strahlendosis einer Steuereinheit, beispielsweise einer EDV-Anlage, zugeführt wird, welche mit diesen Werten die erforderlichen Geschwindigkeiten der einzelnen Fördereinrichtungen bestimmt und überwacht.

16. Verfahren nach einem der vorhergehenden Ansprüche, **dadurch gekennzeichnet, daß** zur Kontrolle während des Produktionsprozesses in definierten Intervallen an einzelnen Gütern, vorzugsweise an der äußeren Oberfläche, Dosimeter angebracht werden, mit deren Hilfe der Bestrahlungsprozeß kontrolliert werden kann.

17. Verfahren nach Anspruch 15 oder 16, **dadurch gekennzeichnet, daß** ein der Steuereinheit zugeordnetes Erkennungssystem die mit Dosimetern versehenen Güter erkennt.

18. Verfahren nach einem der vorhergehenden Ansprüche, **dadurch gekennzeichnet, daß** die aus dem Bestrahlungsprozeß einzelner Güter gewonnen Daten einer Datenverarbeitung zugeführt werden und auf der Basis eines SOLL/ IST-Vergleiches der eingebrachten Dosis die Geschwindigkeit einzelner Anlage-teile, insbesondere des Prozeßförderers, und/oder die Parametereinstellungen des Elektronenbeschleunigersystems geregelt wird.

19. Anlage zur Bestrahlung eines Gutes, insbesondere eines Materials, eines Abfalls, eines Bauteils, eines Lebensmittels, einer Flüssigkeit, eines Gases oder dgl., mit einem einen Prozeßförderer umfassenden Fördersystem für die zu behandelnden Güter, mit einer Quelle zur Erzeugung freier Elektronen, mit einem Beschleunigersystem für die Beschleunigung und Pulsung der freien Elektronen, mit einer Elektronenaustrittsvorrichtung (15), mit einem Bestrahlungsraum, in dem die Güter bestrahlt werden und der von Strahlenschutzwänden umgeben ist, **dadurch gekennzeichnet, daß** das Fördersystem (3) derart ausgestaltet ist, daß das Gut (4) während der Bestrahlung eine aus einer Vertikal- und Drehbewegung zusammengesetzte Bewegung ausführt.

20. Anlage nach Anspruch 19, **dadurch gekennzeichnet, daß** der Bestrahlungsraum (1) auf einem Niveau angeordnet ist, das sich vom Niveau, auf dem sich der Hauptteil des Fördersystems (3) abstützt, unterscheidet und/oder der Bestrahlungsraum (1) unterirdisch angeordnet ist.

21. Anlage nach Anspruch 19 oder 20, **dadurch gekennzeichnet, daß** das Fördersystem (3) außerhalb des Gefahrenbereiches des Beschleunigers für Elektronen aus zwei getrennten Bereichen besteht, wobei der unreine Aufgabebereich durch eine Trennvorrichtung (38), z.B. ein Sperrgitter, eine Sperrwand, vom reinen Abnahmebereich getrennt ist.

22. Anlage nach einem der Ansprüche 19 bis 21, **dadurch gekennzeichnet, daß** der Beschleuniger ein Ringbeschleuniger ist.

23. Anlage nach einem der Ansprüche 19 bis 22, **dadurch gekennzeichnet, daß** die Elektronenaustrittsvorrichtung (15) in Form eines Kreises um das Fördersystem (3) geführt ist.

24. Anlage nach einem der Ansprüche 19 bis 23, **dadurch gekennzeichnet, daß** der Beschleuniger für Elektronen so ausgebildet ist, daß die im Gut (4) deponierte Dosis bis zu einer Eindringtiefe des Elektronenstrahls von 100 cm, bevorzugt 60 cm, weitgehend unabhängig von der Wegstrecke im Gut (4) bzw. in den Gütern (4) ist.

25. Anlage nach einem der Ansprüche 19 bis 24, **dadurch gekennzeichnet, daß** am Fördersystem (3), insbesondere am Prozeßförderer (29), zur Erreichung einer homogenen Dosisverteilung im Gut (4) eine Bewegung in Art und/ oder Geschwindigkeit einstellbar ist, die mindestens 30 %, vorzugsweise 50 %, Überlappung der Pulse des Elektronenstrahls ermöglicht.

26. Anlage nach einem der Ansprüche 19 bis 25, **dadurch gekennzeichnet, daß** der Prozeßförderer (29) derart ausgebildet ist, daß das Gut (4) um einen definierten Winkel, vorzugsweise 360°, gedreht werden kann.

27. Anlage nach einem der Ansprüche 19 bis 26, **dadurch gekennzeichnet, daß** das Fördersystem (3) derart ausgestaltet ist, daß das Gut (4) mit definierter Geschwindigkeit durch den Elektronenstrahl bewegt wird.

28. Anlage nach einem der Ansprüche 19 bis 27, **dadurch gekennzeichnet, daß** zumindest Teile des Fördersystems (3) aus Stetigförderern, beispielsweise einer Rollenbahn und/oder einem Kettenförderer und/oder einem Stabförderer und/oder einem Schwerkraftförderer, bestehen.

29. Anlage nach einem der Ansprüche 19 bis 28, **dadurch gekennzeichnet, daß** das Fördersystem (3) aus einem Aufgabepuffer (24), einem Zuführpuffer (26), einem Prozeßförderer (29), einem Stetigförderer (28), einem Auslagerungspuffer (30), einem Puffer (31), einem Querförderer (25), einer Abnahmebahn und zumindest einem Eckumsetzer mit Hebevorrichtung (46) sowie zumindest einer Antriebsvorrichtung (33) besteht.

30. Anlage nach einem der Ansprüche 19 bis 29, **dadurch gekennzeichnet, daß** entlang des Fördersystems (3) zumindest eine Positionserkennungsvorrichtung (34), beispielsweise eine Lichtschranke (35), vorhanden ist.

31. Anlage nach Anspruch 29 oder 30, **dadurch gekennzeichnet, daß** der Querförderer (25) ein Querverfahrwagen (40) ist.

32. Anlage nach einem der Ansprüche 19 bis 31, **dadurch gekennzeichnet, daß** der Prozeßförderer (29) als Dreh- und/oder Hubtisch ausgebildet ist.

33. Anlage nach einem der Ansprüche 29 bis 32, **dadurch gekennzeichnet, daß** der Auslagerungspuffer (30) eine Schwerkraftbahn ist, vorzugsweise mit flurebenem Staplerabnahmeplatz (53) für die Güter (4) auf den Transportelementen (32).

34. Anlage nach einem der Ansprüche 19 bis 33, **dadurch gekennzeichnet, daß** am Fördersystem (3) zumindest eine Markierungseinrichtung (59) für das Gut (4), beispielsweise ein Etikettenspender, ein Tintenstrahldrucker, eine Vorrichtung zum Anbringen von Mikrochips, angeordnet ist.

35. Anlage nach Anspruch 34, **dadurch gekennzeichnet, daß** die Etiketten für die Güter (4) und/oder Transportelemente (32) mit einem Strich-Code versehen sind.

36. Anlage nach Anspruch 34, **dadurch gekennzeichnet, daß** die verwendeten Mikrochips mit Sendeanlagen, beispielsweise einem IR-Sender ausgerüstet sind.

37. Anlage nach einem der Ansprüche 19 bis 36, **dadurch gekennzeichnet, daß** im Bereich des Fördersystems (3) eine Empfangsstation für IR-Strahlen angeordnet ist.

38. Anlage nach einem der Ansprüche 19 bis 37, **dadurch gekennzeichnet, daß** am Fördersystem zumindest ein Scanner (60), beispielsweise ein Lesegerät, zur Guterfassung angebracht ist.

39. Anlage nach einem der Ansprüche 19 bis 38, **dadurch gekennzeichnet, daß** das Fördersystem (3) zumindest einen Gutstop umfaßt.

40. Anlage nach Anspruch 39, **dadurch gekennzeichnet, daß** vor jeder Richtungsänderung des Fördersystems (3) ein Gutstop angebracht ist.

41. Anlage nach Anspruch 39 oder 40, **dadurch gekennzeichnet, daß** der Gutstop infolge eines Signals der Positionserkennungsvorrichtung (34) aktiviert wird.

42. Anlage nach einem der Ansprüche 19 bis 41, **dadurch gekennzeichnet, daß** das Signal durch Strahlunterbrechung eines Lichtschrankens (35) durch die Güter (4) entsteht.

43. Anlage nach einem der Ansprüche 19 bis 42, **dadurch gekennzeichnet, daß** das Fördersystem (3) eine Zählstation umfaßt.

44. Anlage nach Anspruch 43, **dadurch gekennzeichnet, daß** die Zählstation, vorzugsweise drei, Sensoren umfaßt.

## Claims

1. Method of irradiating an item, in particular a material, an item of waste, a component, a foodstuff, a liquid, a gas, whereby the item, optionally in a packaging and/or on a transport element, is displaced, by means of a conveyor system incorporating a process conveyor, through a beam of moving electrons emitted from at least one electron accelerator, whereby the electrons from an incandescent cathode needed for irradiation purposes are focussed and pulsed in an accelerator unit with waves of a specific, pre-definable frequency and are emitted from the electron-emitting device at a specific frequency and directed onto the item to be irradiated, **characterised in that** the process conveyor describes a vertical and rotary motion during irradiation.

2. Method as claimed in claim 1, **characterised in that** the feed rate of at least a part of the conveyor system, the scanning height and the number of pulses as well as the pulse duration are set up relative to one another such that the pulses of the electron beam overlap by at least 30%, preferably 50%.

3. Method as claimed in claim 1 and/or 2, **characterised in that** the feed rate of the conveyor system is preferably set to fit in with the layout of buffers and the residence time of the item on the process conveyor.

4. Method as claimed in one of the preceding claims, **characterised in that** the item is irradiated alternately or simultaneously from several sides, preferably in a vertical and a horizontal direction relative to the conveyor system.

5. Method as claimed in one of the preceding claims, **characterised in that** radio-chromium film dosimeters are used to measure the dose of energy introduced into the item and alanine transfer dosimeters are used to plot a calibration curve for evaluating the films.

6. Method as claimed in claim 5, **characterised in that** after evaluating the film dosimeter by measuring the intensity of a light beam passing through the film dosimeter in transmission, the detected characteristic values are transferred to an EDP system for processing, storage and control purposes.

7. Method as claimed in one of the preceding claims, **characterised in that** at least one marker, for example a bar code, is applied to the item and/or the packaging of the item, and/or a transport element, which is detected by an identification system, for example a scanner, in particular a reading device.

8. Method as claimed in one of claims 5 to 7, **characterised in that** at least one marker is applied to the film dosimeter for subsequently evaluating, identifying and checking the film dosimeter, in particular a bar code, which is detected by an identification system, for example a scanner, in particular a reading device.

9. Method as claimed in claim 8, **characterised in that** the item is buffered on the basis of the identification system.

10. Method as claimed in one of the preceding claims, **characterised in that** the item or items is or are buffered at two predetermined points of the conveyor system at least, in particular at points at which there is a change of direction.

11. Method as claimed in one of the preceding claims, **characterised in that** the item or items on the transport element is or are fed to the accelerator chamber at intervals of a delivery conveyor device calculated on the basis of the degree of treatment applied.

12. Method as claimed in one of the preceding claims, **characterised in that** the degree to which the item has been treated is detected by a scanner, for example a bar-code reader.

13. Method as claimed in one of the preceding claims, **characterised in that** the item is removed from the processing area by means of a discharge conveyor device, preferably a gravity track, with a flush-aligned discharge position.

14. Method as claimed in one of the preceding claims, **characterised in that** the electron beam strikes the surface of the item in the form of a geometric, preferably almost wavetype curve, which is set by the displacement of the item combined with the displacement of the electron beam.

15. Method as claimed in one of the preceding claims, **characterised in that** a preliminary test is conducted on at least one item to determine the requisite radiation dose by means of dosimeters mounted at a marker point on the item, particularly at those points at which the beam output is particularly low and/or high because of the scanning mode, for example at the centre of the surface and/or in the interior of the item, in the comers of the item, and the calculated requisite radiation dose is applied to a control unit, for example an EDP system, which determines and monitors the requisite speeds of the individual conveyor devices on the basis of these values.

16. Method as claimed in one of the preceding claims, **characterised in that**, for control purposes during the production process, dosimeters are attached to individual items at defined intervals, preferably to the outer surface, by means of which the radiation process can be controlled.

17. Method as claimed in claim 15 or 16, **characterised in that** an identification system co-operating with the control unit detects the items to which dosimeters are attached.

18. Method as claimed in one of the preceding claims, **characterised in that** the data picked up from the individual items during the radiation process is applied to a data processing system and the speed of individual parts of the system, in particular the process conveyor, is regulated and/or the parameter settings of the electron accelerator system are regulated on the basis of a DESIRED/ACTUAL comparison of the dose applied.

19. Plant for irradiating an item, in particular a material, an item of waste, a component, a foodstuff, a liquid, a gas or similar, having a conveyor system incorporating a process conveyor for the items to be processed, having a source for generating free electrons, an accelerator system for accelerating and pulsing the free electrons, with an electron emitter device (15), an irradiation chamber in which the items are irradiated and which is surrounded by walls to protect against radiation, **characterised in that** the conveyor system (3) is set up in such a way that the item (4) is displaced in a movement combining a vertical displacement and a rotary displacement during irradiation.

20. Plant as claimed in claim 19, **characterised in that** the irradiation chamber (1) is arranged on a different level from the level on which the main part of the conveyor system (3) is mounted and/or the irradiation chamber (1) is disposed underground.

21. Plant as claimed in claim 19 or 20, **characterised in that**, outside of the region where the electron accelerator is disposed, the conveyor system (3) consists of two separate regions, the unclean feed region being separated from the clean despatch region by a separator device (38), e.g. a grating, a barrier wall.

22. Plant as claimed in one of claims 19 to 21, **characterised in that** the accelerator is a ring accelerator.

23. Plant as claimed in one of claims 19 to 22, **characterised in that** the electron-emitting device (15) describes a circle around the conveyor system (3).

24. Plant as claimed in one of claims 19 to 23, **characterised in that** the electron accelerator is designed so that when the dose is applied, the item (4) is penetrated by the electron beam by a depth of up to 100 cm, preferably 60 cm, and is largely independent of the travel path in the item (4) or items (4).

25. Plant as claimed in one of claims 19 to 24, **characterised in that**, in order to distribute the dose uniformly in the item (4) on the conveyor system (3), in particular the process conveyor (29), a displacement can be set in terms of nature and/or speed which overlaps with the pulses of the electron beam by at least 30%, preferably 50%.

26. Plant as claimed in one of claims 19 to 25, **characterised in that** the process conveyor (29) is designed so that the item (4) can be rotated by a defined angle, preferably 360°.

27. Plant as claimed in one of claims 19 to 26, **characterised in that** the conveyor system (3) is designed so that the item (4) can be displaced through the electron beam at a defmed speed.

28. Plant as claimed in one of claims 19 to 27, **characterised in that** at least parts of the conveyor system (3) are continuous conveyors, for example a roller track and/or a chain conveyor and/or a bar conveyor and/or a gravity conveyor.

29. Plant as claimed in one of claims 19 to 28, **characterised in that** the conveyor system (3) consists of a feed buffer (24), a delivery buffer (26), a process conveyor (29), a continuous conveyor (28), a switch buffer (30), a buffer (31), a transverse conveyor (25), a despatch track and at least one corner unit with a lifting device (46) and at least one drive device (33).

30. Plant as claimed in one of claims 19 to 29, **characterised in that** at least one position sensor (34), for example a light sensor (35), is provided along the conveyor system (3).

31. Plant as claimed in claim 29 or 30, **characterised in that** the transverse conveyor (25) is a transverse carriage (40).

32. Plant as claimed in one of claims 19 to 31, **characterised in that** the process conveyor (29) is designed as a rotary and/or lifting table.

33. Plant as claimed in one of claims 29 to 32, **characterised in that** the switch buffer (30) is a gravity track, preferably with a flush-aligned stack despatch position (53) for the items (4) on the transport elements (32).

34. Plant as claimed in one of claims 19 to 33, **characterised in that** at least one marker device (59) for the item (4), for example a label dispenser, an ink jet printer, a device for applying microchips or similar, is provided, at least on the conveyor system (3).

35. Plant as claimed in claim 34, **characterised in that** the labels for the items (4) and/or the transport elements (32) are provided with a bar code.

36. Plant as claimed in claim 34, **characterised in that** the microchips used are fitted with transmitters, for example an IR transmitter.

37. Plant as claimed in one of claims 19 to 36, **characterised in that** a receiver station for IR rays is provided in the region of the conveyor system (3).

38. Plant as claimed in one of claims 19 to 37, **characterised in that** at least one scanner (60), for example a reader or similar, is mounted on the conveyor system to detect the items.

39. Plant as claimed in one of claims 19 to 38, **characterised in that** the conveyor system (3) has at least one stop.

40. Plant as claimed in claim 39, **characterised in that** a stop is provided before each change of direction in the conveyor system (3).

41. Plant as claimed in claim 39 or 40, **characterised in that** the stop is activated by means of a signal from the position sensor (34).

42. Plant as claimed in one of claims 19 to 41, **characterised in that** the signal is emitted when the beam from a light sensor (35) is interrupted by the items (4).

43. Plant as claimed in one of claims 19 to 42, **characterised in that** the conveyor system (3) has a counting station.

44. Plant as claimed in claim 43, **characterised in that** the counting system preferably comprises three sensors.

## Revendications

1. Procédé d'irradiation d'un article, en particulier d'un matériau, d'un déchet, d'un composant, d'un aliment, d'un liquide, d'un gaz, où l'article, le cas échéant dans un emballage et/ou sur un élément de transport, est déplacé au moyen d'un système de convoyage comprenant un convoyeur de processus, par un faisceau d'électrons mobiles d'au moins un accélérateur d'électrons dans une chambre d'irradiation, où les électrons requis pour l'irradiation, sortant d'une cathode incandescente sont focalisés et sont pulsés dans une unité d'accélération avec des ondes d'une fréquence déterminée prédéfinissable et sortent à une fréquence déterminée du dispositif de sortie d'électrons et sont dirigés sur l'article à irradier, **caractérisé en ce que** le convoyeur de processus exécute un déplacement constitué d'un déplacement vertical et de rotation pendant l'irradiation.

2. Procédé selon la revendication 1, **caractérisé en ce que** la vitesse d'avance d'au moins une partie du système de convoyage, la hauteur de balayage et le nombre d'impulsions ainsi que la durée des impulsions sont accordés entre eux de façon que les impulsions du faisceau d'électrons se recouvrent au moins de 30%, de préférence de 50%.

3. Procédé selon la revendication 1 et/ou 2, **caractérisé en ce que** le système d'avancement du système de convoyage, de préférence après le montage de tampons, est adapté à la durée de séjour de l'article au convoyeur de processus.

4. Procédé selon l'une des revendications précédentes, **caractérisé en ce que** l'article est irradié alternativement ou simultanément de plusieurs côtés, de préférence dans une direction verticale respectivement horizontale par rapport au système de convoyage.

5. Procédé selon l'une des revendications précédentes, **caractérisé en ce que** pour la mesure de la dose d'énergie introduite dans l'article, des dosimètres photographiques radio-chromes sont utilisés, et pour l'établissement d'une courbe de calibrage, pour l'évaluation des films, des dosimètres de transfert à alanine sont utilisés.

6. Procédé selon la revendication 5, **caractérisé en ce qu'**après l'évaluation des dosimètres photographiques, par la mesure de l'intensité d'un rayon de lumière traversant le dosimètre photographique, en transmission, les valeurs caractéristiques détectées sont transmises à une installation de traitement de l'information pour le traitement ultérieur, le stockage et le contrôle.

7. Procédé selon l'une des revendications précédentes, **caractérisé en ce qu'**est appliqué à l'article et/ou sur l'emballage de l'article et/ou sur un élément de transport au moins un marquage, par exemple un code à barres, qui est détecté par un système de détection, par exemple un scanner, en particulier un appareil de lecture.

8. Procédé selon l'une des revendications 5 à 7, **caractérisé en ce qu'**au moins un marquage est appliqué au dosimètre photographique pour l'évaluation subséquente, la détection et la vérification du dosimètre photographique, en particulier un code à barres, qui est détecté par un système de détection, par exemple un scanner, en particulier un appareil de lecture.

9. Procédé selon la revendication 8, **caractérisé en ce que** l'article est tamponné après le système de détection.

10. Procédé selon l'une des revendications précédentes, **caractérisé en ce que** l'article respectivement les articles sont tamponnés à au moins deux emplacements prédéterminés du système de convoyage, en particulier aux emplacements auxquels a lieu une modification de la direction de déplacement.

11. Procédé selon l'une des revendications précédentes, **caractérisé en ce que** l'article respectivement les articles sur l'élément de transport sont amenés à des intervalles d'une installation de convoyage d'amenée calculés à partir du degré de traitement à l'enceinte de l'accélérateur.

12. Procédé selon l'une des revendications précédentes, **caractérisé en ce que** le degré de traitement d'un article est détecté par un scanner, par exemple un appareil de lecture de codes à barres.

13. Procédé selon l'une des revendications précédentes, **caractérisé en ce que** l'article est retiré du processus par une installation de convoyage de retrait, de préférence une voie de transport par gravité avec un emplacement de retrait plan au sol.

14. Procédé selon l'une des revendications précédentes, **caractérisé en ce que** le faisceau d'électrons passe sur la surface de l'article sous la forme d'une courbe géométrique, de préférence approximativement en forme d'onde, qui est à déterminer par la combinaison du déplacement de l'article avec le déplacement des faisceaux d'électrons.

15. Procédé selon l'une des revendications précédentes, **caractérisé en ce qu'**on détermine à au moins un article, lors d'un essai préalable, la dose de rayonnement requise au moyen de dosimètres appliqués aux emplacements marquants de l'article, en particulier aux emplacements, où la charge de rayonnement, en raison du mode de balayage, est faible et/ou élevée, par exemple au milieu de la surface et/ou dans l'intérieur de l'article, dans les coins de l'article, et que la dose de rayonnement requise déterminée est transmise à une unité de commande, par exemple une installation de traitement des informations, qui détermine et surveille avec ces valeurs les vitesses requises des installations de convoyage individuelles.

16. Procédé selon l'une des revendications précédentes, **caractérisé en ce que** pour le contrôle pendant le processus de production, à des intervalles définis, des dosimètres sont appliqués aux articles individuels, de préférence à la surface extérieure, à l'aide desquels le processus d'irradiation peut être contrôlé.

17. Procédé selon la revendication 15 ou 16, **caractérisé en ce qu'**un système de détection associé à l'unité de commande détecte les articles munis de dosimètres.

18. Procédé selon l'une des revendications précédentes, **caractérisé en ce que** les données obtenues par le processus d'irradiation d'articles individuels sont transmises à un dispositif de traitement de données, et que sur la base d'une comparaison consigne/réelle de la dose entrée, la vitesse de parties d'installation individuelles, en particulier du convoyeur de processus, et/ou les réglages des paramètres du système de l'accélérateur d'électrons est réglé.

19. Installation d'irradiation d'un article, en particulier d'un matériau, d'un déchet, d'un composant, d'un aliment, d'un liquide, d'un gaz ou analogue avec un système de convoyage comprenant un convoyeur de processus pour les articles à traiter, avec une source de génération d'électrons libres, avec un système d'accélérateur pour l'accélération et la pulsation des électrons libres, avec un dispositif de sortie d'électrons (15), avec une enceinte d'irradiation dans laquelle les articles sont irradiés et qui est entourée par des parois de protection contre les rayons, **caractérisée en ce que** le système de convoyage (3) est réalisé de façon que l'article (4), pendant l'irradiation, exécute un déplacement constitué d'un déplacement vertical et de rotation.

20. Installation selon la revendication 19, **caractérisée en ce que** l'enceinte d'irradiation (1) est disposée sur un niveau différent du niveau sur lequel s'appuie la partie principale du système de convoyage (3) et/ou que l'enceinte d'irradiation (1) est disposée sous le sol.

21. Installation selon la revendication 19 ou 20, **caractérisée en ce que** le système de convoyage (3), à d'extérieur de la zone de danger de l'accélérateur d'électrons, est constitué de deux zones séparées, où la zone de chargement non pure est séparée par un dispositif de séparation (38), par exemple une grille d'arrêt, une paroi d'arrêt, de la zone d'enlèvement pure.

22. Installation selon l'une des revendications 19 à 21, **caractérisée en ce que** l'accélérateur est un accélérateur annulaire.

23. Installation selon l'une des revendications 19 à 22, **caractérisée en ce que** le dispositif de sortie d'électrons (15) est guidé sous la forme d'un cercle autour du système de convoyage (3).

24. Installation selon l'une des revendications 19 à 23, **caractérisée en ce que** l'accélérateur des électrons est réalisé de façon que la dose déposée dans l'article (4) est sensiblement indépendante jusqu'à une profondeur de pénétration du faisceau d'électrons de 100 cm, de préférence de 60 cm, du chemin dans l'article (4) respectivement dans les articles (4).

25. Installation selon l'une des revendications 19 à 24, **caractérisée en ce qu'**au système de convoyage (3), en particulier au convoyeur de processus (29), pour atteindre une répartition homogène de la dose dans l'article (4), un déplacement est réglable selon le type et/ou la vitesse qui permet un chevauchement d'au moins 30%, de préférence de 50%, des impulsions du faisceau d'électrons.

26. Installation selon l'une des revendications 19 à 25, **caractérisée en ce que** le convoyeur de processus (29) est réalisé de façon que l'article (4) peut être amené à tourner selon un angle défini, de préférence sur 360°.

27. Installation selon l'une des revendications 19 à 26, **caractérisée en ce que** le système de convoyage (3) est réalisé de façon que l'article (4) soit déplacé à une vitesse définie à travers le faisceau d'électrons.

28. Installation selon l'une des revendications 19 à 27, **caractérisée en ce qu'**au moins des parties du système de convoyage (3) sont constituées de convoyeurs continus, par exemple d'une voie à rouleaux et/ou d'un convoyeur à chaînes et/ou d'un convoyeur à barreaux et/ou d'un convoyeur par gravité.

29. Installation selon l'une des revendications 19 à 28, **caractérisée en ce que** le système de convoyage (3) est constitué d'un tampon de chargement (24), d'un tampon d'amenée (26), d'un convoyeur de processus (29), d'un convoyeur continu (28), d'un tampon de déplacement (30), d'un tampon (31), d'un convoyeur transversal (25), d'une voie de retrait et d'au moins un organe de transfert d'angle avec dispositif de levage (46) et avec au moins un dispositif d'entraînement (33).

30. Installation selon l'une des revendications 19 à 29, **caractérisée en ce qu'**il existe le long du système de convoyage (3) au moins un dispositif de détection de position (34), par exemple un barrage photoélectrique (35).

31. Installation selon la revendication 29 ou 30, **caractérisée en ce que** le convoyeur transversal (25) est un chariot de déplacement transversal (40).

32. Installation selon l'une des revendications 19 à 31, **caractérisée en ce que** le convoyeur de processus (29) est réalisé comme table rotative et/ou de levage.

33. Installation selon l'une des revendications 29 à 32, **caractérisée en ce que** le tampon de déplacement (30) est une voie par gravité, de préférence avec un emplacement de retrait (53) à chariot élévateur, plan au sol, pour les articles (4) sur les éléments de transport (32).

34. Installation selon l'une des revendications 19 à 33, **caractérisée en ce qu'**il est disposé au système de convoyage (3) au moins une installation de marquage (59) pour l'article (4), par exemple un distributeur d'étiquettes, une imprimante à jet d'encre, un dispositif pour appliquer des micropuces.

35. Installation selon la revendication 34, **caractérisée en ce que** les étiquettes pour les articles (4) et/ou les éléments de transport (32) sont pourvus d'un code à barres.

36. Installation selon la revendication 34, **caractérisée en ce que** les micropuces utilisées sont pourvues d'installations d'émission, par exemple d'un émetteur IR.

37. Installation selon l'une des revendications 19 à 36, **caractérisée en ce qu'**il est disposé au voisinage du système de convoyage (3) un poste de réception des rayons IR.

38. Installation selon l'une des revendications 19 à 37, **caractérisée en ce qu'**il est appliqué au système de convoyage au moins un scanner (60), par exemple un appareil de lecture, pour la détection de l'article.

39. Installation selon l'une des revendications 19 à 38, **caractérisée en ce que** le système de convoyage (3) comprend au moins une butée d'arrêt de l'article.

40. Installation selon la revendication 39, **caractérisée en ce qu'**une butée d'arrêt de l'article est disposée en amont de chaque modification de la direction du système de convoyage (3).

41. Installation selon la revendication 39 ou 40, **caractérisée en ce que** la butée d'arrêt de l'article est activée par suite d'un signal du dispositif de détection de position (34).

42. Installation selon l'une des revendications 19 à 41, **caractérisée en ce que** le signal est produit par une interruption du faisceau d'un barrage photoélectrique (35) par les articles (4).

43. Installation selon l'une des revendications 19 à 42, **caractérisée en ce que** le système de convoyage (3) comporte un poste de comptage.

44. Installation selon la revendication 43, **caractérisée en ce que** le poste de comptage comprend de préférence trois capteurs.
